# EUROPEAN PATENT SPECIFICATION

(11) **EP 3 090 070 B1**
(45) Date of publication and mention of the grant of the patent: **29.04.2020**
(21) Application number: 15701433.3
(22) Date of filing: 02.01.2015
(51) Int. Cl.: C12R 1/01

(54) **COMPOSITIONS AND METHODS FOR BIOLOGICAL PRODUCTION OF AMINO ACIDS IN HYDROGENOTROPHIC MICROORGANISMS**
ZUSAMMENSETZUNGEN UND VERFAHREN ZUR BIOLOGISCHEN HERSTELLUNG VON AMINOSÄUREN IN HYDROGENOTROPHEN MIKROORGANISMEN
COMPOSITIONS ET PROCÉDÉS POUR LA PRODUCTION BIOLOGIQUE D'ACIDE AMINÉS CHEZ DES MICRO-ORGANISMES HYDROGÉNOTROPHES

(30) Priority: 02.01.2014 US 201461923120 P
(43) Date of publication of application: 09.11.2016
(73) Proprietor: Trelys, Inc., Hayward, CA 64545 (US)
(72) Inventor: BRADSHAW, Jill, Oakland, CA 94607 (US); HU, Zhihao, 215600 Jiang Su (CN); KOUBA, Jay, St. Helena, CA 94574 (US)
(74) Representative: Greaves Brewster LLP
(86) International application number: PCT/US2015/010068
(87) International publication number: WO 2015/103497

(56) References cited:
- BECKER J ET AL: "From zero to hero Design-based systems metabolic engineering offor-lysine production", METABOLIC ENGINEERING, ACADEMIC PRESS, US, vol. 13, no. 2, 11 January 2011 (2011-01-11), pages 159-168, XP028186100, ISSN: 1096-7176, DOI: 10.1016/J.YMBEN.2011.01.003 [retrieved on 2011-01-15]
- NAERDAL I ET AL: "Analysis and Manipulation of Aspartate Pathway Genes for L-Lysine Overproduction from Methanol by Bacillus methanolicus", APPLIED AND ENVIRONMENTAL MICROBIOLOGY, vol. 77, no. 17, 1 September 2011 (2011-09-01), pages 6020-6026, XP055183283, ISSN: 0099-2240, DOI: 10.1128/AEM.05093-11
- LIU Y ET AL: "Methanococci Use the Diaminopimelate Aminotransferase (DapL) Pathway for Lysine Biosynthesis", JOURNAL OF BACTERIOLOGY, vol. 192, no. 13, 1 July 2010 (2010-07-01) , pages 3304-3310, XP055183292, ISSN: 0021-9193, DOI: 10.1128/JB.00172-10
- GRAHAM D E ET AL: "Methanogens with pseudomurein use diaminopimelate aminotransferase in lysine biosynthesis", FEBS LETTERS, ELSEVIER, AMSTERDAM, NL, vol. 582, no. 9, 16 April 2008 (2008-04-16), pages 1369-1374, XP022587133, ISSN: 0014-5793, DOI: 10.1016/J.FEBSLET.2008.03.021 [retrieved on 2008-03-25]
- CHEN Z ET AL: "Coevolutionary Analysis Enabled Rational Deregulation of Allosteric Enzyme Inhibition in Corynebacterium glutamicum for Lysine Production", APPLIED AND ENVIRONMENTAL MICROBIOLOGY, vol. 77, no. 13, 1 July 2011 (2011-07-01), pages 4352-4360, XP055183284, ISSN: 0099-2240, DOI: 10.1128/AEM.02912-10
- LIU X ET AL: "The Structural Basis for Allosteric Inhibition of a Threonine-sensitive Aspartokinase", JOURNAL OF BIOLOGICAL CHEMISTRY, vol. 283, no. 23, 6 June 2008 (2008-06-06) , pages 16216-16225, XP055183297, ISSN: 0021-9258, DOI: 10.1074/jbc.M800760200

## Description

### BACKGROUND

Amino acids gained commercial significance shortly after the turn of the century with the discovery of the flavor-enhancing quality of glutamic acid and the marketing of monosodium glutamate in Japan. Several amino acids, including those not synthesized by animals or humans (referred to as essential amino acids), are used as additives for animal feed, as food supplements for humans, and in intravenous solutions for sustaining gravely ill patients. The U.S. market for amino acids alone represents 20% of the global market, which is predicted to exceed $2 billion by 2016, with more than half of the market being for animal feed supplements.

Amino acids for commercial use are generally produced in four ways: extraction from natural sources, chemical synthesis, fermentation, and enzymatic catalysis. Although still used for certain amino acids, extraction is now of limited importance. Chemical synthesis can be carried out on a very large scale, but the reactions typically produce racemic mixtures and this requires resolution and recovery of the desired enantiomer. One exception is essential amino acid methionine, which has been manufactured synthetically as a racemate (DL-methionine) from the starting materials acrolein, hydrocyanic acid, methyl mercaptan, and ammonia, and has been marketed as a feed additive for more than 50 years. Using the methionine racemate is feasible because animals have enzymes to convert the non-natural D-form methionine into the beneficial L-form. In contrast, no such comparable enzyme system for conversion of the D-form exists for other amino acids, so amino acids other than methionine must be produced in the pure L-form. Nonetheless, these processes either require harsh production environments or result in environmentally detrimental byproducts. Today, the most preferred methods for amino acid production are fermentation and enzymatic catalysis.

The discovery of the soil bacterium, *Corynebacterium glutamicum,* which is capable of producing the flavor-enhancer L-glutamic acid with high productivity from sugar, paved the way for the success of fermentation in amino acid production (Kinoshita et al., J. Gen. Appl. Microbiol. 3:193, 1957). The rapid development of the amino acid market over the last 30 years is due in no small part to advances in fermentation technology and strain improvement of amino acid producing microorganisms, like *C. glutamicum* or *E. coli,* which has enabled industrial-scale production of many amino acids (Ikeda M (2003) Amino acid production processes. In: Scheper T, Faurie R, Thommel J (eds) Advances in biochemical engineering/biotechnology, vol 79. Springer, New York, pp 1-35; Leuchtenberger et al., Appl. Microbiol. Biotechnol. 69:1, 2005). But, the fermentation market is dominated far and away by natural alcohol and synthetic alcohol products, particularly ethanol. The carbohydrate feedstock used in amino acid production is used directly in animal feed and in biofuel production. Growth in demand for animal products and biofuels has increased the cost of carbohydrate feedstocks, which in turn increases the cost of producing amino acids.

Given the high price of feedstocks, there is a need in the art for alternative and improved methods for biologically producing amino acids in a cost-effective manner. The present disclosure meets such needs, and further provides other related advantages.

### BRIEF SUMMARY

In certain aspects, the present disclosure provides a non-natural hydrogenotrophic microorganism, wherein the non-natural hydrogenotrophic microorganism expresses a deregulated aspartokinase activity, and wherein the non-natural hydrogenotrophic microorganism metabolizes a H₂/COₓ substrate to produce one or more aspartate pathway amino acids at a higher level than a parent hydrogenotrophic microorganism, as defined in the claims.

In still further aspects, the present disclosure provides a method for producing an aspartate pathway amino acid, comprising culturing non-natural hydrogenotrophic microorganisms according to this disclosure for a time sufficient to produce one or more aspartate pathway amino acids.

In additional aspects, the present disclosure provides a system for producing an aspartate pathway amino acid, comprising a source of gas comprising a H₂/COₓ substrate; a bioreactor comprising any one or more of the non-natural hydrogenotrophic microorganisms of this disclosure; and a connector disposed between the gas source and the bioreactor to allow flow of the gas into the bioreactor; wherein the non-natural hydrogenotrophic microorganism metabolizes the H₂/COₓ substrate to overproduce one or more aspartate pathway amino acids (such as lysine, threonine, methionine) as compared to a parent hydrogenotrophic microorganism.

### BRIEF DESCRIPTION OF THE DRAWINGS

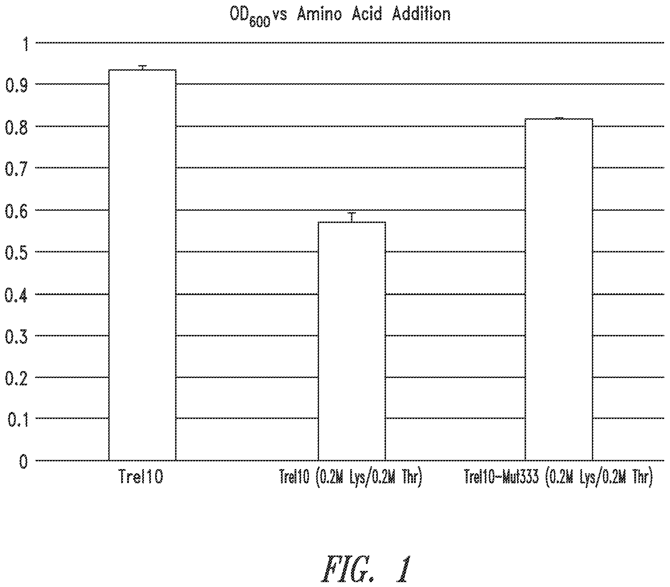

**Figure 1** shows the deregulated growth rate of a *Methanococcus maripaludis lysC* mutant Trel10-Mut333, which has a G333R mutation (corresponding to LysC amino acid position G277 of *Corynebacterium glutamicum* ATCC 13032), in presence of lysine and threonine (OD₆₀₀ was measured after incubation at 37°C for 72 hours).

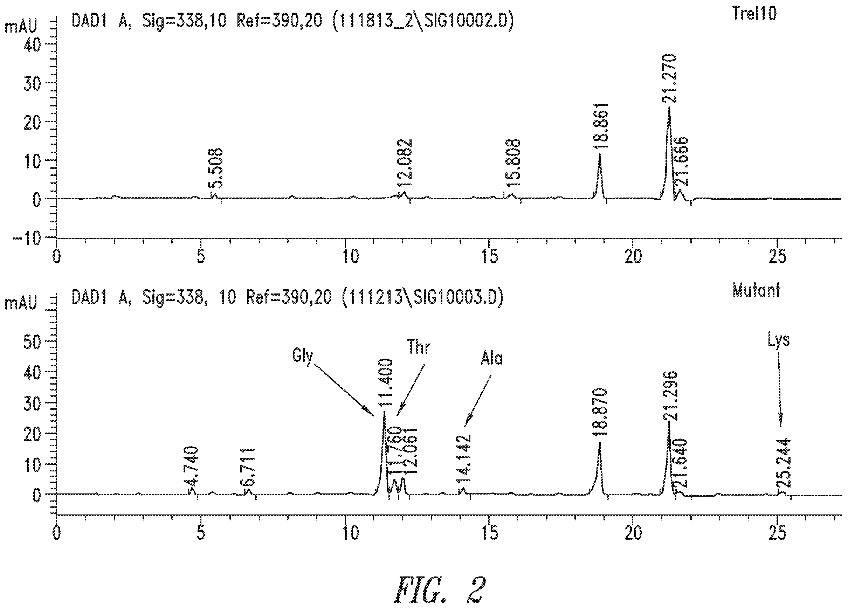

**Figure 2** shows an HPLC graph of aspartate pathway amino acid production by *Methanococcus maripaludis* having a mutated endogenous aspartokinase enzyme. The production profile in the mutant as compared to the parent strain was alanine (5 mg/L), lysine (8 mg/L), threonine (21 mg/L), and glycine (78 mg/L).

### DETAILED DESCRIPTION

The instant disclosure provides compositions and methods for biologically utilizing or converting gas into useful compositions, such as high-value molecules (*e.g*., amino acids), biological material (*e.g*., animal feed), or a combination thereof. For example, synthesis gas can be fed to hydrogenotrophic microorganisms to generate one or more different aspartate pathway amino acids. This approach allows for the use of hydrogenotrophic archaea or bacteria as a new host system to utilize or convert a feedstock comprising hydrogen gas and a carbon oxide (*e.g*., CO, CO₂) into lysine, glycine, threonine, methionine, or any combination thereof.

By way of background, aspartate pathway amino acids - such as lysine, threonine, and methionine - share several enzymes in their biosynthetic pathways, one or more of which are subject to feedback regulation, repression of gene expression, or both. For example, aspartokinase, the first committed enzyme involved in directing carbon flux into the biosynthesis of these industrially important amino acids, is allosterically inhibited from phosphorylating aspartate by threonine and lysine in *Corynebacterium glutamicum* (Sano and Shiio, J. Gen. Appl. Microbiol. 16:373, 1970; Yoshida et al., J. Mol. Biol. 368:521, 2007). Homoserine dehydrogenase is the first committed enzyme in the threonine/methionine biosynthetic pathways, but this enzyme competes for aspartyl semialdehyde with the first enzyme committed to the lysine biosynthetic pathway, dihydrodipicolinate synthase. Control of the carbon flux toward threonine/methionine or lysine will depend on which enzyme obtains the substrate - for example, in *Corynebacterium,* homoserine dehydrogenase activity is inhibited by threonine and expression is repressed by methionine, but dihydrodipicolinate synthase is not affected by lysine levels. Similarly, homoserine *O*-succinyltransferase is subject to feedback regulation by methionine and S-adenosylmethionine (Born and Blanchard, Biochem. 38:14416, 1999).

The present disclosure provides compositions and methods for metabolic engineering (*e.g*., altering genes, altering gene expression, altering gene expression regulation) of hydrogenotrophic microorganisms to redirect precursor and metabolic fluxes toward producing higher levels of particular amino acids (*e.g*., lysine, threonine, methionine) as compared to wild-type or parent organisms. In certain aspects, the present disclosure provides compositions, methods, and systems for producing amino acids, comprising use of a modified hydrogenotrophic microorganism, wherein the modified hydrogenotrophic microorganism metabolizes a H₂/COₓ substrate to produce one or more aspartate pathway amino acids at a higher level than a parent hydrogenotrophic microorganism.

Prior to setting forth this disclosure in more detail, it may be helpful to an understanding thereof to provide definitions of certain terms to be used herein. Additional definitions are set forth throughout this disclosure.

In the present description, any concentration range, percentage range, ratio range, or integer range is to be understood to include the value of any integer within the recited range and, when appropriate, fractions thereof (such as one tenth and one hundredth of an integer), unless otherwise indicated. Also, any number range recited herein relating to any physical feature, such as polymer subunits, size or thickness, are to be understood to include any integer within the recited range, unless otherwise indicated. As used herein, the term "about" means ± 20% of the indicated range, value, or structure, unless otherwise indicated. The term "consisting essentially of' limits the scope of a claim to the specified materials or steps, or to those that do not materially affect the basic and novel characteristics of the claimed invention. It should be understood that the terms "a" and "an" as used herein refer to "one or more" of the enumerated components. The use of the alternative (*e.g*., "or") should be understood to mean either one, both, or any combination thereof of the alternatives. As used herein, the terms "include," "have" and "comprise" are used synonymously, which terms and variants thereof are intended to be construed as non-limiting.

As used herein, "aspartate pathway amino acids" or "aspartate family of amino acids" refers to one or more amino acids synthesized from aspartate, including lysine, threonine, methionine, homoserine, isoleucine and glycine. While steps in the biosynthetic pathways for each of the aspartate family of amino acids may branch or diverge, they all begin with the phosphorylation of aspartate by an aspartate kinase (also referred to as an aspartokinase). In certain embodiments, an aspartate kinase in the biosynthetic pathway of the aspartate family of amino acids is subject to feedback inhibition by one or more of lysine, threonine, and methionine. Furthermore, reference to a protein or enzyme from a "pathway for biosynthesis of an aspartate family amino acid" may mean, for example, a lysine biosynthetic pathway enzyme, a glycine biosynthetic pathway enzyme, a threonine biosynthetic pathway enzyme, a methionine biosynthetic pathway enzyme, or any combination thereof.

As used herein, "hydrogenotroph" or "hydrogenotrophic" refers to a microorganism capable of consuming H₂, oxidizing H₂, or converting H₂ into another compound as part of its metabolism. In certain embodiments, a hydrogenotroph may be an obligate or facultative hydrogenotroph, an obligate or facultative anaerobe, or any combination thereof. For example, a facultative hydrogenotroph may grow in the presence or absence of hydrogen as an energy source, and may use one or more various carbon sources, such as carbohydrates, acetate, formate, methanol, methylamines, or carbon oxide (*e.g.,* an Acetogen, *Clostridium,* may grow in the absence of H₂ and use acetate as both an energy and carbon source). Exemplary hydrogenotrophs include Methanogens, Acetogens, Knall-gas bacteria, or the like.

As used herein, a "H₂/COₓ substrate" or "H₂/COₓ feedstock" refers to a mixture of hydrogen (H2) with carbon dioxide (CO₂) or carbon monoxide (CO) or both, which may also include various other components, such as ammonia (NH₃), hydrocarbons (*e.g*., methane (CH₄)), CO₂, CO, formaldehyde (CH₂O), hydrogen sulfide (H₂S), carbonyl sulfide, (COS), hydrogen cyanide (HCN), water vapor, inert gases, or other gases.

As used herein, "synthesis gas" or "syngas" refers to a mixture of carbon monoxide and hydrogen, which may be produced, for example, by steam reforming, dry or CO₂ reforming, autothermal reforming, catalytic partial oxidation or partial oxidation of natural gas or liquid hydrocarbons, within hydrogen synthesis, within ammonia synthesis, within methanol synthesis, by steelmaking, or by gasification of coal, biomass or waste. In certain embodiments, syngas can be further conditioned by a water-gas shift reaction. Syngas may also include methane, CO₂, H₂S, or other gases in smaller quantities relative to CO and H₂.

As used herein, the term "host" refers to a cell or microorganism (*e.g*., archaea, bacteria) that may be genetically modified by mutation or with an exogenous nucleic acid molecule to produce a polypeptide of interest (*e.g*., aspartokinase). In certain embodiments, a host cell may optionally already possess other genetic modifications that confer desired properties related or unrelated to the mutated or exogenous polypeptide being expressed (*e.g*., deregulation). For example, a host cell may possess or be altered to possess genetic modifications conferring additional or enhanced carbon flux activity into the aspartate pathway amino acids, reduced production of competing amino acids, high growth, tolerance of contaminants or particular culture conditions, ability to metabolize additional carbon substrates, or ability to synthesize desirable products or intermediates.

As used herein, the term "methanogen" or "methanogenic archaea" refers to a microorganism capable of producing methane under anoxic conditions using hydrogen gas and any of various one or two carbon substrates (*e.g*., carbon dioxide, acetate, formic acid, formaldehyde, carbon monoxide, methanol, methyl amines (*e.g*., methylamine, dimethylamine, trimethylamine, or the like)). It is understood in the art that bacteria are not archaea and archaea are not bacteria. As used herein, methanogenic archaea may be "obligate hydrogenotrophs," which require hydrogen gas to produce methane. Methanogenic archaea may be "facultative hydrogenotrophs," which are able to produce methane in the absence of hydrogen gas. Furthermore, methanogenic archaea may be mesophilic, thermophilic or hyperthermophilic.

As used herein, "biomass" refers to organic material having a biological origin, which may include whole cells, lysed cells, extracellular material, product produced or a portion thereof, or the like. For example, the material harvested from a cultured microorganism (*e.g*., bacterial or archaeal culture) may be considered the biomass, which can include secreted products or can be the secreted products.

As used herein, "nucleic acid molecule," also known as a polynucleotide, refers to a polymeric compound comprised of covalently linked subunits called nucleotides. Nucleic acid molecules include polyribonucleic acid (RNA), polydeoxyribonucleic acid (DNA), both of which may be single or double stranded. DNA includes cDNA, genomic DNA, synthetic DNA, semi-synthetic DNA, or the like.

As used herein, the term "endogenous" or "native" refers to a gene, protein, compound or activity that is normally present in a host cell. Moreover, a gene, protein or activity that is mutated, overexpressed, shuffled, duplicated or otherwise altered as compared to a parent gene, protein or activity is still considered to be endogenous or native to that particular host cell. For example, an endogenous control sequence from a first gene (*e.g.*, promoter, translational attenuation sequences) may be used to alter or regulate expression of a second native gene or nucleic acid molecule, wherein the expression or regulation of the second native gene or nucleic acid molecule differs from normal expression or regulation in a parent cell.

As used herein, "heterologous" or "exogenous" nucleic acid molecule, construct or sequence refers to a nucleic acid molecule or portion of a nucleic acid molecule that is not native to a host cell, but may be homologous to a nucleic acid molecule or portion of a nucleic acid molecule from the host cell. The source of the heterologous or exogenous nucleic acid molecule, construct or sequence may be from a different genus or species. In certain embodiments, a heterologous or exogenous nucleic acid molecule is added (*i.e.,* not endogenous or native) to a host cell or host genome by, for example, conjugation, transformation, transfection, electroporation, or the like, wherein the added molecule may integrate into the host genome or exist as extra-chromosomal genetic material (*e.g*., as a plasmid or other form of self-replicating vector), and may be present in multiple copies. In addition, "heterologous" refers to a non-native enzyme, protein or other activity encoded by an exogenous nucleic acid molecule introduced into the host cell, even if the host cell encodes a homologous protein or activity.

The term "homologous" or "homolog" refers to a molecule or activity found in or derived from a host cell, species or strain. For example, a heterologous or exogenous nucleic acid molecule may be homologous to a native host cell gene, and may optionally have an altered expression level, a different sequence, an altered activity, or any combination thereof.

As used herein, the term "non-natural" refers to an organism, microorganism, cell, nucleic acid molecule, or vector that includes at least one genetic alteration that differs from a wild-type or parent cell or molecule. For example, non-natural may refer to a microorganism or cell that has been altered (*e.g*., site-specific or random mutants, including spontaneous mutants) such that the expression of an endogenous nucleic acid molecule or gene, or the activity of a gene product, has been altered (*e.g*., increased, decreased, deregulated, activated, derepressed, repressed) as compared to a wild-type or parent microorganism. Such modifications include, for example, those in non-coding regulatory regions that alter expression of a gene or operon. A "non-natural" organism, microorganism, or cell can include recombinant organisms, microorganisms, or cells.

As used herein, the term "recombinant" refers to a microorganism, cell, nucleic acid molecule, or vector that has been modified by introduction of an exogenous nucleic acid molecule, or refers to a microorganism or cell that has been altered such that expression of an endogenous nucleic acid molecule or gene is controlled, deregulated or constitutive, where such alterations or modifications may be introduced by genetic engineering. Genetic alterations may include, for example, modifications introducing nucleic acid molecules (which may include an expression control element, such as a promoter) encoding one or more proteins or enzymes, or other nucleic acid molecule additions, deletions, substitutions, or other functional disruption of or addition to a cell's genetic material. Exemplary modifications include those in coding regions or functional fragments thereof of heterologous or homologous polypeptides from a reference or parent microorganism. In certain embodiments, an organism, microorganism, or cell of this disclosure is a non-natural organism, microorganism, or cell and a recombinant organism, microorganism, or cell. For example, a non-natural hydrogenotrophic microorganism that expresses or overexpresses a deregulated endogenous enzyme (*e.g*., aspartokinase, homoserine *O*-acetyltransferase) may also contain one or more exogenous or heterologous nucleic acid molecules that are expressed or overexpressed to produce certain enzyme acitvities involved in biosynthesis of aspartate pathway amino acids (*e.g*., asparate semialdehyde dehydrogenase, dihydrodipicolinate synthase, dihydrodipicolinate reductase, LL-diaminopimelate aminotransferase, diaminopimelate decarboxylase).

As used herein, "transformation" refers to the introduction of a nucleic acid molecule (*e.g*., exogenous or heterologous nucleic acid molecule) into a host cell. The transformed host cell may carry the exogenous or heterologous nucleic acid molecule extra-chromosomally or integrated in the chromosome. Integration into a host cell genome and self-replicating vectors generally result in genetically stable inheritance of the transformed nucleic acid molecule. Host cells containing the transformed nucleic acids are referred to as "recombinant" or "genetically engineered" or "transformed" or "transgenic" cells (*e.g*., bacteria, archaea).

As used herein, the term "deregulated" refers to reduced or increased expression of a gene product, or reduced or increased activity of a gene product (*e.g.,* a protein, enzyme) as compared to gene expression or activity, respectively, in a parent or wild-type microorganism. For example, a microorganism can be genetically manipulated (*e.g*., mutated, genetically engineered) to increase or decrease the expression of a gene product or to increase or reduce the activity of the gene product over that of a parent or wild-type microorganism prior to manipulation. In certain embodiments, a target gene is mutated such that the expressed gene product has increased activity. For example, the coding region of a target gene may be altered so that the expressed gene product has increased activity, the copy number of the target gene may be increased to increase activity, a target gene may be overexpressed to increase activity, or any combination thereof. In other embodiments, a target gene is mutated such that the expressed gene product has a reduced, minimal or non-detectable response to feedback inhibition (*e.g*., an amino acid biosynthetic enzyme, such as aspartokinase, homoserine *O-*acetyltransferase or homoserine *O*-transsuccinyltransferase, is deregulated in the presence of one or more feedback inhibitors lysine, threonine and methionine). In further embodiments, a target gene is mutated such that the gene has a reduced, minimal or non-detectable response to repression of expression (*e.g*., an amino acid biosynthetic enzyme, such as homoserine dehydrogenase, is deregulated in the presence of feedback co-repressor methionine). Alternatively, a microorganism may be identified, for example, under selective pressure to have any one or more of the above-noted genetic alterations (*e.g*., spontaneous mutants). A deregulated gene or gene product of any of the aforementioned embodiments may be a spontaneous, induced or engineered mutant or variant.

As used herein, the term "overexpressed" refers to a level of gene expression or gene product in a non-natural or recombinant microorganism that is greater than the level of gene expression or gene product found in a parent or wild-type microorganism when grown under the same conditions. In certain embodiments, overexpression may occur at the transcriptional level, translational level, or both, which may be due to altered regulatory control (*e.g*., use of a strong promoter) or an increase in copy number or both.

The "percent identity" between two or more nucleic acid sequences is a function of the number of identical positions shared by the sequences (*i.e.,* % identity = number of identical positions/total number of positions x 100), taking into account the number of gaps, and the length of each gap that needs to be introduced to optimize alignment of two or more sequences. The comparison of sequences and determination of percent identity between two or more sequences can be accomplished using a mathematical algorithm, such as BLAST and Gapped BLAST programs at their default parameters (*e.g.,* Altschul et al., J. Mol. Biol. 215:403, 1990; see also BLASTN at www.ncbi.nlm.nih.gov/BLAST).

A "conservative substitution" is recognized in the art as a substitution of one amino acid for another amino acid that has similar properties. Exemplary conservative substitutions are well known in the art (*see, e.g.,* WO 97/09433 at page 10; Lehninger, Biochemistry, 2nd Edition; Worth Publishers, Inc. NY, NY, pp.71-77, 1975; Lewin, Genes IV, Oxford University Press, NY and Cell Press, Cambridge, MA, p. 8, 1990).

"Inhibit" or "inhibited," as used herein, refers to an alteration, reduction, down regulation or abrogation, directly or indirectly, in the expression of a target gene or in the activity of a target molecule (*e.g*., phosphoenolpyruvate synthase) relative to a control, endogenous or reference molecule, wherein the alteration, reduction, down regulation or abrogation is statistically, biologically, industrially, or clinically significant. For example, an inhibited, inactivated or reduced activity biosynthetic enzyme (*e.g*., genetically altered) may possess 35%, 30%, 25%, 20%, 15%, 14%, 13%, 12%, 11%, 10%, 9%, 8%, 7%, 6%, 5%, 4%, 3%, 2%, 1%, or less activity as compared to a wild-type or parent enzyme.

As used herein, the term "derivative" refers to a modification of a compound by chemical or biological means, with or without an enzyme, which modified compound is structurally similar to a parent compound and (actually or theoretically) derivable from that parent compound. A derivative may have different chemical, biological or physical properties from the parent compound, such as being more hydrophilic or having altered reactivity as compared to the parent compound. Derivatization (*i.e.,* modification) may involve substitution of one or more moieties within the molecule (*e.g.,* a change in functional group). For example, a hydrogen may be substituted with a halogen, such as fluorine or chlorine, or a hydroxyl group (-OH) may be replaced with a carboxylic acid moiety (-COOH). Other exemplary derivatizations include glycosylation, alkylation, acylation, acetylation, ubiqutination, esterification, and amidation.

The term "derivative" also refers to all solvates, for example, hydrates or adducts (*e.g*., adducts with alcohols), active metabolites, and salts of a parent compound. The type of salt depends on the nature of the moieties within the compound. For example, acidic groups, such as carboxylic acid groups, can form alkali metal salts or alkaline earth metal salts (*e.g*., sodium salts, potassium salts, magnesium salts, calcium salts, and also salts with physiologically tolerable quaternary ammonium ions and acid addition salts with ammonia and physiologically tolerable organic amines such as, for example, triethylamine, ethanolamine or tris-(2-hydroxyethyl)amine). Basic groups can form acid addition salts with, for example, inorganic acids such as hydrochloric acid, sulfuric acid or phosphoric acid, or with organic carboxylic acids or sulfonic acids such as acetic acid, citric acid, lactic acid, benzoic acid, maleic acid, fumaric acid, tartaric acid, methanesulfonic acid or p-toluenesulfonic acid. Compounds that simultaneously contain a basic group and an acidic group, for example, a carboxyl group in addition to basic nitrogen atoms, can be present as zwitterions. Salts can be obtained by customary methods known to those skilled in the art, for example, by combining a compound with an inorganic or organic acid or base in a solvent or diluent, or from other salts by cation exchange or anion exchange.

### Hydrogenotrophic Microorganisms - Host Cells

A parent or starting hydrogenotrophic microorganism of the instant disclosure may be a wild-type (natural) strain, a mutated (non-natural) strain (*e.g*., increased growth rate, deregulated or derepressed biosynthetic enzyme), or a recombinant strain, each of which may be further modified to produce one or more amino acids (*e.g.,* lysine, glycine, threonine, methionine) at a higher level than the parent hydrogenotrophic microorganism.

The present disclosure provides hydrogenotrophic microorganisms that are methanogenic archaea, namely *Methanococcus maripaludis* or *Methanococcus vannielii.*

### Hydrogenotrophic Microorganisms - Non-Natural and Recombinant

The hydrogenotrophic microorganisms of this disclosure can be genetically manipulated (e.g., non-natural), recombinantly modified or combinations thereof to knock-out, reduce, express or over-express polypeptides of interest, which results in recombinant microorganisms useful for converting (*e.g*., utilizing, converting, assimilating, oxidizing, reducing) various components of a H₂/COₓ substrate into other useful compounds or compositions, such as amino acids or feed.

Genetic manipulation to generate non-natural hydrogenotrophic microorganisms can include random (*e.g*., chemically-induced, spontaneous) or site-directed mutagenesis (*e.g*., of one or more gene targets), alteration of regulatory sequences or sites associated with expression of one or more gene targets (*e.g*., by removing strong, weak, inducible, repressible, or multiple promoters, or by replacing such promoters with promoters having different properties), changing the chromosomal location of one or more gene targets, altering nucleic acid sequences adjacent to one or more gene targets (such as a ribosome binding site or transcription terminator), decreasing or increasing the copy number of one or more gene targets, modifying regulatory proteins, repressors, suppressors, enhancers, transcriptional activators or the like involved in transcription of one or more gene targets or translation of one or more gene products, or any other method of deregulating expression of one or more gene targets (including the use of antisense nucleic acid molecules, short interfering nucleic acid molecules, or other methods to knock-out or block expression of a target protein).

In certain embodiments, a genetic manipulation comprises one or more spontaneous mutations that result in a non-natural hydrogenotrophic microorganism. Such spontaneous mutants can be identified, for example, by placing microorganisms under a particular selective pressure where only a mutant with the desired phenotype will grow (*e.g*., absence of a particular amino acid or toxin in the growth medium, presence of an antibiotic, absence of a particular metabolite, or the like).

Variation in codon usage bias has been observed across different species of bacteria and archaea, which may affect recombinant protein expression in a heterologous host (Sharp et al., Nucl. Acids Res. 33:1141, 2005; Emery and Sharp, Biol. Lett. 7:131, 2011). In certain embodiments, nucleic acid molecules (*e.g.,* nucleic acids encoding aspartate pathway amino acid biosynthetic enzymes) may be codon optimized prior to introduction into a host cell as described herein to improve or maximize protein expression. Codon optimization refers to the alteration of codon sequence in genes or coding regions at the nucleic acid molecule level to reflect a more common codon usage of a host cell without altering the amino acid encoded by the codon. Codon optimization methods for gene expression in heterologous hosts have been previously described (*see, e.g.,* Welch et al., Methods Enzymol. 498:43, 2011; Henry and Sharp, Mol. Biol. Evol. 24:10, 2007; U.S. Patent Publication No. 2011/0111413).

In further embodiments, endogenous or exogenous nucleic acid molecules encoding a biosynthetic enzyme of this disclosure may be altered, such as having the amino acid sequence changed from wild-type. Each variant polypeptide generated by these methods will retain at least 50% activity (preferably 100% or more activity) and have a polypeptide sequence that is at least 90%, at least 91%, at least 92%, at least 93%, at least 94%, at least 95%, at least 96%, at least 97%, at least 98%, at least 99% identical, or 100% identical to a reference or parental wild-type polypeptide sequence. In certain embodiments, variant polypeptides will include at least one amino acid substitution (*e.g*., at least 1, 2, 3, 5, 6, 7, 8, 9 or 10 or more or up to 20, 25, or 30 substitutions) or no more than a particular number of amino acid substitutions (*e.g*., no more than 1, 2, 3, 5, 6, 7, 8, 9, 10, 15, 20, 25, or 30 substitutions) at one or more predetermined positions relative to a reference or parental wild-type enzyme, provided that a variant retains an activity of interest (*e.g*., carboxylase, decarboxylase, dehydrogenase, epimerase, kinase, lyase, reductase, synthase).

As noted above, the first committed enzyme in the biosynthesis of aspartate pathway amino acids is aspartokinase, which may be subject to feedback regulation by one or more of lysine, threonine and methionine. For example, *E. coli* has three aspartokinase isozymes - two are bifunctional with aspartokinase and homoserine dehydrogenase activity, which are referred to as aspartokinase I-homoserine dehydrogenase I (AK/HD-I; *thrA*) and aspartokinase II-homoserine dehydrogenase II (AK/HD-II; *metL*)*,* and the other has aspartokinase activity alone, which is referred to as aspartokinase III (AK-III; *lysC*)*.* The AK/HD-I is subject to feedback regulation by threonine (as well as repression of expression by threonine and leucine), while AK/HD-II is subject to feedback regulation by methionine only and AK-III is subject to feedback regulation by lysine only (*see* Patte et al., Biochim. Biophys. Acta 136:245, 1967; Theze et al., J. Bacteriol. 117:133, 1974). In contrast, the *Corynebacterium glutamicum* aspartokinase is feedback inhibited by both lysine and threonine (Sano and Shiio, 1970; Yoshida *et al.,* 2007). Other enzymes involved in the biosynthesis of aspartate pathway amino acids are also subject to feedback inhibition, such as homoserine *O*-acetyltransferase, homoserine *O*-transsuccinyltransferase, dihydrodipicolinate synthase.

Hence, the present disclosure provides hydrogenotrophic microorganisms having deregulated enzymes involved in the biosynthesis of aspartate pathway amino acids, such as an aspartokinase, homoserine *O*-acetyltransferase, homoserine O-transsuccinyltransferase or dihydrodipicolinate synthase, which activities may be endogenous, exogenous, or both. In certain embodiments, an aspartokinase may optionally have a conjugated homoserine dehydrogenase activity. Methods for identifying feedback resistant mutants are known in the art - for example, microorganisms capable of growing in the presence of toxic amino acid analogs, such as lysine analog S-2-aminoethyl-L-cysteine (AEC) or methionine analog DL-ethionine, are considered to be feedback resistant to the amino acid corresponding to the toxic analog (*see, e.g.,* Shiio et al., Agric. Bioi. Chem. 54:3275, 1990; Kumar and Gomes, Biotechnology Advances 23:41-61, 2005).

In certain aspects, the present disclosure provides a non-natural methanogenic archaea selected from,
(a) a non-natural *Methanococcus maripaludis* expressing a deregulated, aspartokinase mutant enzyme encoded by a recombinant *Methanococcus LysC* gene, wherein the aspartokinase mutant is resistant to feedback inhibition by lysine and/or threonine and has a mutation at the residue corresponding to residue G277, S302 or G359 in the aspartokinase encoded by lysC of Corynebacterium glutamicum ATCC 13032, or
(b) a non-natural *Methanococcus vannielii* expressing a deregulated, aspartokinase mutant enzyme encoded by a recombinant *Methanococcus LysC* gene that has a mutation at residue K380 or F339 and is resistant to feedback inhibition by lysine and/or threonine; and
wherein the non-natural methanogenic archaea (i) is not susceptible to growth inhibition by lysine and/or threonine, and (ii) metabolizes a H2/COx substrate to produce one or more aspartate pathway amino acids at a higher level than a parent methanogenic archaea.

In further embodiments, a deregulated endogenous aspartokinase activity is encoded by a mutant *lysC* gene comprising a mutation at a threonine binding site, a lysine binding site, a lysine and threonine binding site, a site other than a lysine or threonine binding site, or any combination thereof. In certain embodiments, a deregulated endogenous aspartokinase activity is encoded by a mutant *thrA* gene comprising a mutation at a threonine binding site. In other embodiments, a deregulated endogenous aspartokinase activity is encoded by a mutant *metL* gene comprising a mutation at a methionine binding site.

When referring to *lysC* feedback resistant mutants of this disclosure, reference is made to the residue numbering that corresponds to the amino acid positions of the *Corynebacterium glutamicum* ATCC 13032 LysC protein (GenBank Accession No. CAF18822.1). Exemplary threonine binding site mutations include residue 1272, D274, G277, E278, A279, D294, Q298, N372, N374, 1375, or any combination thereof. Exemplary lysine binding site mutations include residue 1291, 1293, D294, T361, S381, E382, or any combination thereof. An exemplary lysine and threonine binding site mutation is at residue D294. Exemplary mutations at a site other than a lysine and threonine binding site include residue F283, N299, S301, S302, T308, T311, T336, G359, F364, M365, T380, R384, S386, or any combination thereof. Any one or more of the aforementioned mutations may be included in an aspartokinase of this disclosure, provided that the aspartokinase polypeptide retains its kinase activity.

In order for biosynthesis of aspartate pathway amino acids to occur efficiently, a certain amount of carbon flux must flow through the pathway. One way to boost or enhance the production of aspartate pathway amino acids is to maximize the carbon flux into this pathway, as provided by this disclosure.

In further embodiments, a non-natural hydrogenotrophic microorganism expressing a deregulated endogenous aspartokinase activity also has reduced phosphoenolpyruvate synthase activity, increased pyruvate kinase activity, or both. In still further embodiments, a non-natural hydrogenotrophic microorganism expressing a deregulated endogenous aspartokinase activity also has increased pyruvate carboxylase activity, increased pyruvate synthase, increased acetyl-CoA synthase, increased aspartate aminotransferase activity, or any combination thereof. In each of these embodiments, the non-natural hydrogenotrophic microorganism metabolizes a H₂/COₓ substrate to produce one or more aspartate pathway amino acids at a higher level than a parent hydrogenotrophic microorganism.

As noted herein, several of the biosynthetic enzymes that produce the aspartate pathway amino acids are subject to feedback regulation (*e.g*., aspartokinase, homoserine *O*-acetyltransferase, homoserine *O*-transsuccinyltransferase, dihydrodipicolinate synthase), the genes that encode these enzymes are subject to repression (*e.g*., homoserine dehydrogenase), or both (*e.g*., dihydrodipicolinate synthase). Hence, production of aspartate pathway amino acids can be improved by relieving the regulation, repression, or both, as provided by this disclosure.

In further embodiments, a non-natural hydrogenotrophic microorganism expressing a deregulated endogenous aspartokinase activity may also have one or more other deregulated and/or derepressed polypeptides from one or more pathways for biosynthesis of an aspartate family amino acid. A non-natural hydrogenotrophic microorganism expressing a deregulated endogenous aspartokinase activity may also have a homoserine dehydrogenase, homoserine kinase, dihydrodipicolinate synthase, homoserine *O*-acetyltransferase (*e.g., metA*)*, O*-succinylhomoserine lyase (*e.g., metB*)*,* or any combination thereof that are derepressed, deregulated, or both, wherein the derepression or deregulation or both are due to one or more spontaneous mutations, random mutations, site specific mutations, or any combination thereof.

In addition to overproducing one or more aspartate pathway amino acids, it would be advantageous to avoid extraction or isolation of the produced amino acids. Accordingly, the present disclosure provides methods for enhanced production of one or more aspartate pathway amino acids in the culture medium where isolation or purification methods are simplified.

Sometimes, simply overexpressing one or more biosynthetic enzymes that produce aspartate family amino acids will be useful in the hydrogenotrophic microorganisms of the instant disclosure.

In further embodiments, a non-natural hydrogenotrophic microorganism expressing a deregulated endogenous aspartokinase activity also overexpresses a polypeptide from one or more pathways for biosynthesis of an aspartate family amino acid. In certain further embodiments, a non-natural hydrogenotrophic microorganism expressing a deregulated endogenous aspartokinase activity also overexpresses an asparate semialdehyde dehydrogenase, dihydrodipicolinate synthase, dihydrodipicolinate reductase (also known as dihydropicolinate reductase), LL-diaminopimelate aminotransferase, diaminopimelate decarboxylase, or any combination thereof; or an asparate semialdehyde dehydrogenase (*e.g.,* encoded by an *asd* or variant thereof), dihydrodipicolinate synthase (*e.g.,* encoded by a *dapA* or variant thereof), dihydrodipicolinate reductase (*e.g.,* encoded by a *dapB* or variant thereof), LL-diaminopimelate aminotransferase (*e.g.,* encoded by a *dapL* or variant thereof), and optionally a diaminopimelate decarboxylase (*e.g.,* encoded by a *lysA* or variant thereof); or overexpresses a homoserine dehydrogenase, homoserine kinase or both; or overexpresses a homoserine dehydrogenase, serine acetyltransferase or both; or overexpresses homoserine *O*-acetyltransferase, *O*-acetylhomoserine sulfhydrylase or both; or overexpresses a polypeptide having aspartokinase activity.

Recombinant methods for expression of exogenous or heterologous nucleic acids in microbial organisms are well known in the art. Such methods can be found described in, for example, Sambrook et al., Molecular Cloning: A Laboratory Manual, Third Ed., Cold Spring Harbor Laboratory, New York (2001); and Ausubel et al., Current Protocols in Molecular Biology, John Wiley and Sons, Baltimore, MD (1999). Exemplary exogenous proteins or enzymes to be expressed include those involved in lysine biosynthesis (*e.g*., aspartokinase, aspartokinase I-homoserine dehydrogenase I, aspartokinase II-homoserine dehydrogenase II, aspartate semialdehyde dehydrogenase, dihydrodipicolinate synthase, dihydrodipicolinate reductase [also known as dihydropicolinate reductase], LL-diaminopimelate aminotransferase, meso-diaminopimelate dehydrogenase, tetrahydrodipicolinate succinylase, tetrahydrodipicolinate acetyltransferase, *N*-succinyl diaminopimelate aminotransferase, acetyl-diaminopimelate aminotransferase, *N*-succinyl diaminopimelate desuccinylase, *N*-acetyl diaminopimelate acetylase, diaminopimelate epimerase, diaminopimelate decarboxylase, or any combination thereof), threonine biosynthesis (*e.g*., aspartokinase, aspartate semialdehyde dehydrogenase, homoserine dehydrogenase, homoserine kinase, threonine synthase, or any combination thereof), methionine biosynthesis (*e.g*., aspartokinase, aspartate semialdehyde dehydrogenase, homoserine dehydrogenase, homoserine *O*-acetyltransferase, homoserine *O*-transsuccinyltransferase, *O*-succinylhomoserine lyase, cystathionine γ-synthase, cystathionine β-lyase, *O*-acetylhomoserine sulfhydrylase, homocysteine *S*-methyltransferase, methionine synthase (cobalamin dependent or independent), or any combination thereof), or enzymes affecting carbon flux into the aspartate family of amino acids biosynthetic pathway (*e.g*., pyruvate kinase, pyruvate carboxylase, pyruvate synthase, acetyl-CoA synthase, aspartate aminotransferase, or any combination thereof). Genetic modifications to nucleic acid molecules encoding enzymes, or functional fragments thereof, can confer a biochemical or metabolic capability to a recombinant cell that is altered from its naturally occurring state.

Any of the hydrogenotrophic microorganisms of this disclosure may be transformed to comprise at least one exogenous nucleic acid to provide the host with a new or enhanced activity (*e.g*., enzymatic activity) or may be genetically modified to remove or substantially reduce an endogenous gene function using any of a variety of methods known in the art. Genetic tools for transfer and expression of heterologous nucleic acid molecules in hydrogenotrophic microorganisms, such as methanogenic archaea, is known in the art (*see, e.g.,* Rother et al., Curr. Opin. Microbiol. 8:745, 2005; Leigh et al., FEMS Microbiol. Rev. 35:577, 2011). For example, tools are available for DNA delivery (Dodsworth et al., Appl. Environ. Microb. 76:5644, 2010; Metcalf et al., Proc. Natl. Acad. Sci. U. S. A. 94:2626, 1997), for shuttle vectors (Gardner and Whitman, Genetics 152:1439, 1999; Metcalf *et al.,* 1997), for regulated expression of heterologous genes (Lie and Leigh, J. Bacteriol. 184:5301, 2002; Chaban et al., Mol. Microbiol. 66:596, 2007; Guss et al., Archaea 2:193, 2008), and for knock-in or knock-out genetic exchange (Moore and Leigh, J. Bacteriol. 187:972, 2005; Pritchett et al., Appl. Environ. Microb. 70:1425, 2004). Therefore, various methods for inactivating, knocking-out, or deleting endogenous gene function in hydrogenotrophic microorganisms may be used.

In certain embodiments, promoters, codon optimization, or both can be used for high, constitutive expression of exogenous nucleic acid molecules encoding amino acid biosynthesis pathway enzymes in host hydrogenotrophic microorganisms. Regulated expression of an exogenous nucleic acid molecule in a host hydrogenotrophic microorganism (*e.g*., methanogenic archaea) may also be utilized. In certain embodiments, regulated expression of exogenous nucleic acid molecules encoding amino acid biosynthesis enzymes may be desirable to optimize growth rate of the non-natural hydrogenotrophic microorganisms. Controlled expression of nucleic acid molecules encoding amino acid biosynthesis enzymes for response to the presence of a H₂/COₓ substrate may improve growth based on the variety of different sources or ratios of H₂/COₓ substrate available.

As described herein, more than one heterologous or exogenous nucleic acid molecule can be introduced into a host cell as separate nucleic acid molecules, as a plurality of individually controlled genes, as a polycistronic nucleic acid molecule, as a single nucleic acid molecule encoding a fusion protein, or any combination thereof. For example, as disclosed herein, a H₂/COₓ metabolizing microorganism can be modified to express two or more heterologous or exogenous nucleic acid molecules encoding desired biosynthetic enzymes of the aspartate family of amino acids (*e.g.,* aspartokinase, aspartokinase I-homoserine dehydrogenase I, aspartokinase II-homoserine dehydrogenase II, aspartate semialdehyde dehydrogenase, dihydrodipicolinate synthase, LL-diaminopimelate aminotransferase, meso-diaminopimelate dehydrogenase, homoserine dehydrogenase, homoserine kinase, homoserine synthase, homoserine *O*-acetyltransferase, homoserine *O*-transsuccinyltransferase, *O*-succinylhomoserine lyase, cystathionine γ-synthase). When two or more exogenous nucleic acid molecules are introduced into a host H₂/COₓ metabolizing microorganism, it is understood that the two more exogenous nucleic acid molecules can be introduced as a single nucleic acid molecule (*e.g*., on a single vector), on separate vectors, integrated into the host chromosome at a single site or multiple sites. The number of referenced heterologous nucleic acid molecules or protein activities refers to the number of encoding nucleic acid molecules or the number of protein activities, not the number of separate nucleic acid molecules introduced into a host cell.

For example, a hydrogenotrophic microorganism (such as a methanogen) can be recombinantly transformed to produce a polypeptide capable of utilizing, converting or metabolizing a H₂/COₓ substrate (*e.g*., H₂ with CO₂, CO, or both) into an aspartate pathway amino acid at a higher level than a parent microorganism, such as lysine, threonine, or methionine.

By way of background, at least four different lysine biosynthetic pathways exist in Archaea and bacteria, and it is not uncommon for more than one pathway to be present, although all four are generally not present simultaneously. Different steps in the pathway are catalyzed by various enzymes and, therefore, each of these may be over-expressed to increase the amount of enzyme to drive the production of lysine. Nucleic acid molecules encoding enzymes required for these lysine biosynthetic pathways may also be recombinantly added to a hydrogenotrophic microorganism lacking such enzymes. Finally, (a) steps that might compete with the pathway leading to lysine production can be attenuated, blocked or knocked-out, (b) steps that might enhance lysine production (*e.g*., shift carbon flux to lysine biosynthesis) can be altered, or (c) both may be changed, in order to maximize the amount of lysine produced.

A first pathway for lysine biosynthesis includes the following enzymes: aspartokinase, aspartate semialdehyde dehydrogenase, dihydrodipicolinate synthase, dihydrodipicolinate reductase, LL-diaminopimelate aminotransferase, diaminopimelate epimerase, and diaminopimelate decarboxylase. A second pathway for lysine biosynthesis includes the following enzymes: aspartokinase, aspartate semialdehyde dehydrogenase, dihydrodipicolinate synthase, dihydrodipicolinate reductase, meso-diaminopimelate dehydrogenase, and diaminopimelate decarboxylase. A third pathway for lysine biosynthesis includes the following enzymes: aspartokinase, aspartate semialdehyde dehydrogenase, dihydrodipicolinate synthase, dihydrodipicolinate reductase, tetrahydrodipicolinate succinylase, *N*-succinyl diaminopimelate aminotransferase, *N*-succinyl diaminopimelate desuccinylase, diaminopimelate epimerase, and diaminopimelate decarboxylase. A fourth pathway for lysine biosynthesis includes the following enzymes: aspartokinase, aspartate semialdehyde dehydrogenase, dihydrodipicolinate synthase, dihydrodipicolinate reductase, tetrahydrodipicolinate acetyltransferase, acetyl-diaminopimelate aminotransferase, *N*-acetyl diaminopimelate acetylase, diaminopimelate epimerase, and diaminopimelate decarboxylase.

A non-natural hydrogenotrophic microorganism expressing a deregulated endogenous aspartokinase activity may further comprise a first exogenous nucleic acid molecule encoding one or more polypeptides from a lysine biosynthetic pathway, and the non-natural hydrogenotrophic microorganism produces lysine at a higher level than the parent hydrogenotrophic microorganism, as described herein. In further embodiments, the one or more polypeptides from a lysine biosynthetic pathway are aspartokinase, aspartate semialdehyde dehydrogenase, dihydrodipicolinate synthase, dihydrodipicolinate reductase, LL-diaminopimelate aminotransferase, diaminopimelate epimerase, diaminopimelate decarboxylase, or any combination thereof. In other embodiments, the one or more polypeptides from a lysine biosynthetic pathway are aspartokinase, aspartate semialdehyde dehydrogenase, dihydrodipicolinate synthase, dihydrodipicolinate reductase, meso-diaminopimelate dehydrogenase, diaminopimelate decarboxylase, or any combination thereof.

In particular embodiments, a first exogenous nucleic acid molecule encodes a dihydrodipicolinate synthase, wherein the dihydrodipicolinate synthase is optionally overexpressed. In further embodiments, a first exogenous nucleic acid molecule encodes an asparate semialdehyde dehydrogenase (*e.g.,* encoded by an *asd* or variant thereof), a dihydrodipicolinate synthase (*e.g.,* encoded by a *dapA* or variant thereof), a dihydrodipicolinate reductase (*e.g.,* encoded by a *dapB* or variant thereof), an LL-diaminopimelate aminotransferase (*e.g.,* encoded by a *dapL* or variant thereof), and a diaminopimelate decarboxylase (*e.g.,* encoded by a *lysA* or variant thereof).

A non-natural hydrogenotrophic microorganism expressing a deregulated endogenous aspartokinase activity may further comprise a first exogenous nucleic acid molecule encoding one or more polypeptides from a lysine biosynthetic pathway, and further have (a) one or more threonine biosynthetic pathway polypeptides that are knocked out or have reduced activity, (b) one or more methionine biosynthetic pathway polypeptides that are knocked out or have reduced activity, (c) one or more glycine biosynthetic pathway polypeptides that are knocked out or have reduced activity, (d) one or more lysine degradation pathway polypeptides (*e.g., cadA*) that are knocked out or have reduced activity, or (e) a combination thereof. In certain embodiments, a homoserine dehydrogenase gene is knocked out or encodes a reduced activity homoserine dehydrogenase mutant.

In any of the aforementioned non-natural hydrogenotrophic microorganisms, the exogenous nucleic acid molecule is integrated into the genome or the exogenous nucleic acid molecule is in a self-replicating vector. Additionally, in any of the aforementioned non-natural hydrogenotrophic microorganisms, the non-natural hydrogenotrophic microorganism is a homoserine auxotroph, methionine auxotroph, threonine auxotroph, glycine auxotroph, or any combination thereof.

A non-natural hydrogenotrophic microorganism expressing a deregulated endogenous aspartokinase activity may further comprise a first exogenous nucleic acid molecule encoding one or more polypeptides from a threonine biosynthetic pathway, and the non-natural hydrogenotrophic microorganism produces threonine at a higher level than the parent hydrogenotrophic microorganism, as described herein. In further embodiments, the one or more polypeptides from a threonine biosynthetic pathway are selected from aspartokinase, aspartate semialdehyde dehydrogenase, homoserine dehydrogenase, homoserine kinase, threonine synthase, or any combination thereof. In particular embodiments, a first exogenous nucleic acid molecule encodes a homoserine dehydrogenase, a homoserine kinase, or both, wherein the homoserine dehydrogenase, homoserine kinase, or both are optionally overexpressed, deregulated, or both.

In some embodiments, a non-natural hydrogenotrophic microorganism expressing a deregulated endogenous aspartokinase activity further comprises a first exogenous nucleic acid molecule encoding one or more polypeptides from a threonine biosynthetic pathway, and further has (a) one or more lysine biosynthetic pathway polypeptides that are knocked out or have reduced activity, (b) one or more methionine biosynthetic pathway polypeptides that are knocked out or have reduced activity, (c) one or more isoleucine biosynthetic pathway polypeptides that are knocked out or have reduced activity, (d) one or more glycine biosynthetic pathway polypeptides that are knocked out or have reduced activity, (e) one or more threonine degradation pathway polypeptides (*e.g., tdh*) that are knocked out or have reduced activity, or (f) any combination thereof. In certain embodiments, the lysine or isoleucine biosynthetic pathway nucleic acid molecule that encodes a homoserine *O*-acetyltransferase, a dihydrodipicolinate synthase, a threonine dehydratase, a threonine aldolase, a serine hydroxymethyl transferase, or any combination thereof is knocked out or encodes a reduced activity homoserine *O*-acetyltransferase mutant, dihydrodipicolinate synthase mutant, threonine dehydratase mutant, threonine aldolase mutant, serine hydroxymethyl transferase mutant, or any combination thereof.

In any of the aforementioned non-natural hydrogenotrophic microorganisms, the exogenous nucleic acid molecule is integrated into the genome or the exogenous nucleic acid molecule is in a self-replicating vector. Additionally, in any of the aforementioned non-natural hydrogenotrophic microorganisms, the non-natural hydrogenotrophic microorganism is a lysine auxotroph, methionine auxotroph, isoleucine auxotroph, glycine auxotroph, or any combination thereof.

In even further embodiments, a non-natural hydrogenotrophic microorganism expressing a deregulated endogenous aspartokinase activity further comprises a first exogenous nucleic acid molecule encoding one or more polypeptides from a methionine biosynthetic pathway, and the non-natural hydrogenotrophic microorganism produces methionine at a higher level than the parent hydrogenotrophic microorganism, as described herein. In further embodiments, the one or more polypeptides from a methionine biosynthetic pathway are selected from aspartokinase, aspartate semialdehyde dehydrogenase, homoserine dehydrogenase, homoserine *O*-acetyltransferase, homoserine *O*-transsuccinyltransferase (*e.g., metA*)*, O*-succinylhomoserine lyase (*e.g., metB*)*,* cystathionine γ-synthase, cystathionine β-lyase, *O*-acetylhomoserine sulfhydrylase, homocysteine S-methyltransferase, methionine synthase (cobalamin dependent or independent), or any combination thereof. In particular embodiments, a first exogenous nucleic acid molecule encodes a homoserine dehydrogenase, a serine acetyltransferase, or both, wherein the homoserine dehydrogenase, serine acetyltransferase, or both are optionally overexpressed, deregulated, or both.

In certain other embodiments, a first exogenous nucleic acid molecule encodes a homoserine *O*-acetyltransferase, an *O*-acetylhomoserine sulfhydrylase, or both, wherein the homoserine *O*-acetyltransferase, *O*-acetylhomoserine sulfhydrylase, or both are optionally overexpressed, deregulated, or both are overexpressed. In still other embodiments, a first exogenous nucleic acid molecule encodes an *E. coli* ThrA (AK/HD-I), an *E. coli* MetL (AK/HD-II), a homoserine *O*-acetyltransferase, an *O-*acetylhomoserine sulfhydrylase, or combinations thereof, wherein the AK/HD-I, AK/HD-II, homoserine *O*-acetyltransferase, *O*-acetylhomoserine sulfhydrylase, or combinations thereof are optionally overexpressed, deregulated, or both are overexpressed. In particular embodiments, the *E. coli* ThrA (AK/HD-I) is a deregulated mutant, wherein the AK/HD-I is mutated at any one or more of amino acid positions G330, S345, S352, and G433.

In some embodiments, a non-natural hydrogenotrophic microorganism expressing a deregulated endogenous aspartokinase activity further comprises a first exogenous nucleic acid molecule encoding one or more polypeptides from a methionine biosynthetic pathway, and further has (a) one or more lysine biosynthetic pathway polypeptides that are knocked out or have reduced activity, (b) one or more threonine biosynthetic pathway polypeptides that are knocked out or have reduced activity, (c) one or more glycine biosynthetic pathway polypeptides that are knocked out or have reduced activity, (d) one or more methionine degradation pathway polypeptides (*e*.*g*., *metK*) that are knocked out or have reduced activity, or (e) any combination thereof. In certain embodiments, the nucleic acid molecule that encodes a dihydrodipicolinate synthase, a threonine dehydratase, a serine hydroxymethyl transferase, or any combination thereof are knocked out or encode a reduced activity.

In any of the aforementioned non-natural hydrogenotrophic microorganisms, the exogenous nucleic acid molecule is integrated into the genome or the exogenous nucleic acid molecule is in a self-replicating vector. Additionally, in any of the aforementioned non-natural hydrogenotrophic microorganisms, the non-natural hydrogenotrophic microorganism is a lysine auxotroph, threonine auxotroph, glycine auxotroph, or any combination thereof.

In certain embodiments, hydrogenotrophic microorganisms as described herein may be engineered to express or overproduce aspartokinase (EC 2.7.2.4) or dihydrodipicolinate synthase (EC 4.3.3.7), and optionally engineered to also express or overproduce LL-diaminopimelate aminotransferase (EC 2.6.1.83), meso-diaminopimelate dehydrogenase (EC 1.4.1.16), or both. In certain embodiments, hydrogenotrophic microorganisms are engineered to express or overproduce a dihydrodipicolinate synthase (EC 4.3.3.7), a dihydrodipicolinate reductase (EC 1.3.1.26), and an LL-diaminopimelate aminotransferase (EC 2.6.1.83). In further embodiments, hydrogenotrophic microorganisms are engineered to express or overproduce an asparate semialdehyde dehydrogenase (EC 1.2.1.11), a dihydrodipicolinate synthase (EC 4.3.3.7), a dihydrodipicolinate reductase (EC 1.3.1.26), an LL-diaminopimelate aminotransferase (EC 2.6.1.83), and optionally a diaminopimelate decarboxylase (EC 4.1.1.20). In any of these embodiments, the hydrogenotrophic microorganisms may further optionally express or overproduce an asparate semialdehyde dehydrogenase (EC 1.2.1.11) in order to produce, for example, detectable or excess lysine. In any of these embodiments, the hydrogenotrophic microorganisms may encode a mutated LysC (EC 2.7.2.4) or a mutated AK/HD-I (EC 2.7.2.4 / EC 1.1.1.3) (*e.g.,* nucleic acid position G1297A, which corresponds to amino acid change G433R) that is resistant to feedback inhibition by lysine.

For example, to express or overproduce aspartokinase, one or more genes from *E. coli* (*thrA*)*, E. coli* (*metL*)*, E. coli* (*lysC*)*, Corynebacterium glutamicum* (*lysC*)*,* or *Methanococcus maripaludis* (*lysC*) can be introduced into and expressed or overexpressed in a hydrogenotrophic microorganism (*e.g*., methanogen), thereby producing or overproducing exogenous aspartokinase or a functional fragment thereof. In certain embodiments, aspartokinase polypeptides for use in the compositions and methods disclosed herein are from *Corynebacterium glutamicum* ATCC 13032 (Genbank Accession No. CAF18822.1), *Methanococcus maripaludis* S2 (Genbank Accession No. CAF30573.1), *Methanocella conradii* HZ254 (Genbank Accession No. AFD00291.1), *Methanobrevibacter ruminantium* M1 (Genbank Accession No. ADC47522.1), *E. coli* K-12 substr. W3110 thrA (GenBank Accession No. BAB96579.2); *E. coli* K-12 substr. W3110 metL (GenBank Accession No. BAE77370.1); *E. coli K-12* substr. W3110 lysC (GenBank Accession No. BAE78026.1).

In some embodiments, an aspartokinase amino acid sequence or a functional fragment thereof is based on the *thrA, metL,* or *lysC* amino acid sequence from *E. coli* K-12 substr. W3110 and is at least at least 75%, at least 80% identical, at least 85% identical, at least 90% identical, at least 91% identical, at least 92% identical, at least 93% identical, at least 94% identical, at least 95% identical, at least 96% identical, at least 97% identical, at least 98% identical, or at least 99% identical to the sequence set forth in Genbank Accession No. BAB96579.2, BAE77370.1 or BAE78026.1, respectively, or a functional fragment thereof. In other embodiments, a recombinantly encoded aspartokinase has an amino acid sequence that is codon optimized for a host cell, or is identical to a sequence as set forth in Genbank Accession Nos. BAB96579.2, BAE77370.1 or BAE78026.1, or comprises a consensus sequence of these aspartokinases or comprises a consensus sequence of a plurality of known aspartokinase polypeptides. In particular embodiments, an aspartokinase amino acid sequence is an *E. coli* ThrA protein (GenBank Accession No. BAB96579.2) mutated at any one or more of amino acid positions G330, S345, S352 and G433 (to, for example, aspartate, phenylalanine, or arginine), or is an *E. coli* LysC protein (GenBank Accession No. BAE78026.1) mutated at amino acid position T342 (to, for example, isoleucine).

In certain embodiments, an aspartokinase amino acid sequence or a functional fragment thereof is based on the amino acid sequence of *Corynebacterium glutamicum* ATCC 13032 or *Methanococcus maripaludis* S2 and is at least at least 75%, at least 80% identical, at least 85% identical, at least 90% identical, at least 91% identical, at least 92% identical, at least 93% identical, at least 94% identical, at least 95% identical, at least 96% identical, at least 97% identical, at least 98% identical, or at least 99% identical to the sequence set forth in Genbank Accession No. CAF 18822.1 or CAF30573.1, respectively, or a functional fragment thereof. In other embodiments, a recombinantly encoded aspartokinase has an amino acid sequence that is codon optimized for a host cell or is identical to a sequence as set forth in Genbank Accession Nos. CAF 18822.1, CAF30573.1, AFD00291.1, or ADC47522.1, or comprises a consensus sequence of these aspartokinases or comprises a consensus sequence of a plurality of known aspartokinase polypeptides.

For example, to express or overproduce dihydrodipicolinate synthase, one or more genes from *Corynebacterium glutamicum* (*DapA*)*,* or *Methanococcus maripaludis* (*DapA*) can be introduced into and expressed or overexpressed in a hydrogenotrophic microorganism (*e.g*., methanogen), thereby producing or overproducing exogenous dihydrodipicolinate synthase or a functional fragment thereof. In certain embodiments, dihydrodipicolinate synthase polypeptides for use in the compositions and methods disclosed herein are from *Corynebacterium glutamicum* ATCC 13032 (Genbank Accession No. CAF20312.1), *Methanococcus maripaludis* S2 (Genbank Accession No. CAF30132.1), *Methanocella conradii* HZ254 (Genbank Accession No. AFC99231.1), or *Methanobrevibacter ruminantium* M1 (Genbank Accession No. ADC47521.1).

In certain embodiments, a dihydrodipicolinate synthase amino acid sequence or a functional fragment thereof is based on the amino acid sequence of *Corynebacterium glutamicum* ATCC 13032 or *Methanococcus maripaludis* S2 and is at least at least 75%, at least 80% identical, at least 85% identical, at least 90% identical, at least 91% identical, at least 92% identical, at least 93% identical, at least 94% identical, at least 95% identical, at least 96% identical, at least 97% identical, at least 98% identical, or at least 99% identical to the sequence set forth in Genbank Accession No. CAF20312.1 or CAF30132.1, respectively, or a functional fragment thereof. In other embodiments, a recombinantly encoded dihydrodipicolinate synthase has an amino acid sequence that is codon optimized for a host cell or is identical to a sequence as set forth in Genbank Accession Nos. CAF20312.1, CAF30132.1, AFC99231.1, or ADC47521.1, or comprises a consensus sequence of these dihydrodipicolinate synthases or comprises a consensus sequence of a plurality of known dihydrodipicolinate synthase polypeptides.

For example, to express or overproduce LL-diaminopimelate aminotransferase, one or more genes from *Methanococcus maripaludis* or *Methanobrevibacter ruminantium* (*DapL*) can be introduced into and expressed or overexpressed in a hydrogenotrophic microorganism (*e.*g., methanogen), thereby producing or overproducing exogenous LL-diaminopimelate aminotransferase or a functional fragment thereof. In certain embodiments, LL-diaminopimelate aminotransferase polypeptides for use in the compositions and methods disclosed herein are from *Methanococcus maripaludis* S2 (Genbank Accession No. Q6LX26.1) or *Methanobrevibacter ruminantium* M1 (Genbank Accession No. ADC46792.1).

In certain embodiments, a LL-diaminopimelate aminotransferase amino acid sequence or a functional fragment thereof is based on the amino acid sequence of *Methanococcus maripaludis* S2 or *Methanobrevibacter ruminantium* M1 and is at least at least 75%, at least 80% identical, at least 85% identical, at least 90% identical, at least 91% identical, at least 92% identical, at least 93% identical, at least 94% identical, at least 95% identical, at least 96% identical, at least 97% identical, at least 98% identical, or at least 99% identical to the sequence set forth in Genbank Accession No. Q6LX26.1 or ADC46792.1, respectively, or a functional fragment thereof. In other embodiments, a recombinantly encoded LL-diaminopimelate aminotransferase has an amino acid sequence that is codon optimized for a host cell or is identical to a sequence as set forth in Genbank Accession Nos. Q6LX26.1 or ADC46792.1, or comprises a consensus sequence of these LL-diaminopimelate aminotransferases or comprises a consensus sequence of a plurality of known LL-diaminopimelate aminotransferase polypeptides.

For example, to express or overproduce meso-diaminopimelate dehydrogenase, one or more genes from *Corynebacterium glutamicum* (*lysC*)*,* or *Methanococcus maripaludis* (*lysC*) can be introduced into and expressed or overexpressed in a hydrogenotrophic microorganism (*e.g*., methanogen), thereby producing or overproducing exogenous meso-diaminopimelate dehydrogenase or a functional fragment thereof. In certain embodiments, meso-diaminopimelate dehydrogenase polypeptides for use in the compositions and methods disclosed herein are from *Corynebacterium glutamicum* ATCC 13032 (Genbank Accession No. CAF21279.1) or *Clostridium tetani* E88 (Genbank Accession No. AA036992.1).

In certain embodiments, a meso-diaminopimelate dehydrogenase amino acid sequence or a functional fragment thereof is based on the amino acid sequence of *Corynebacterium glutamicum* ATCC 13032 or *Clostridium tetani* E88 and is at least at least 75%, at least 80% identical, at least 85% identical, at least 90% identical, at least 91% identical, at least 92% identical, at least 93% identical, at least 94% identical, at least 95% identical, at least 96% identical, at least 97% identical, at least 98% identical, or at least 99% identical to the sequence set forth in Genbank Accession No. CAF21279.1 or AA036992.1, respectively, or a functional fragment thereof. In other embodiments, a recombinantly encoded meso-diaminopimelate dehydrogenase has an amino acid sequence that is codon optimized for a host cell or is identical to a sequence as set forth in Genbank Accession Nos. CAF21279.1 or AA036992.1, or comprises a consensus sequence of these meso-diaminopimelate dehydrogenases or comprises a consensus sequence of a plurality of known meso-diaminopimelate dehydrogenase polypeptides.

In certain embodiments, hydrogenotrophic microorganisms as described herein may be engineered to express or overproduce homoserine dehydrogenase (EC 1.1.1.3), homoserine kinase (EC 2.7.1.39) or threonine synthase (EC 4.2.3.1). In further embodiments, hydrogenotrophic microorganisms as described herein may be engineered to express or overproduce AK/HD-I (EC 2.7.2.4 / EC 1.1.1.3) and homoserine kinase (EC 2.7.1.39) in order to produce, for example, detectable or excess threonine. In still further embodiments, hydrogenotrophic microorganisms as described herein may be engineered to express or overproduce AK/HD-I (EC 2.7.2.4 / EC 1.1.1.3), homoserine kinase (EC 2.7.1.39) and threonine synthase (EC 4.2.3.1) in order to produce, for example, detectable or excess threonine. In any of these embodiments, the hydrogenotrophic microorganisms may further optionally express or overproduce an asparate semialdehyde dehydrogenase (EC 1.2.1.11). In any of these embodiments, the hydrogenotrophic microorganisms may encode a mutated AK/HD-I (EC 2.7.2.4 / EC 1.1.1.3) (*e.g.,* G1297A) that is resistant to feedback inhibition by threonine.

For example, to express or overproduce homoserine dehydrogenase, one or more genes from *Corynebacterium glutamicum* (hom), or *Methanococcus maripaludis* (hom) can be introduced into and expressed or overexpressed in a hydrogenotrophic microorganism (*e.g*., methanogen), thereby producing or overproducing exogenous homoserine dehydrogenase or a functional fragment thereof. In certain embodiments, homoserine dehydrogenase polypeptides for use in the compositions and methods disclosed herein are from *Corynebacterium glutamicum* ATCC 13032 (Genbank Accession No. BAB98576.1), *Methanococcus maripaludis* S2 (Genbank Accession No. CAF31258.1), *Methanocella conradii* HZ254 (Genbank Accession No. AFD00624.1), or *Methanobrevibacter ruminantium* M1 (Genbank Accession No. ADC46990.1).

In certain embodiments, a homoserine dehydrogenase amino acid sequence or a functional fragment thereof is based on the amino acid sequence of *Corynebacterium glutamicum* ATCC 13032 or *Methanococcus maripaludis* S2 and is at least at least 75%, at least 80% identical, at least 85% identical, at least 90% identical, at least 91% identical, at least 92% identical, at least 93% identical, at least 94% identical, at least 95% identical, at least 96% identical, at least 97% identical, at least 98% identical, or at least 99% identical to the sequence set forth in Genbank Accession No. BAB98576.1 or CAF31258.1, respectively, or a functional fragment thereof. In other embodiments, a recombinantly encoded homoserine dehydrogenase has an amino acid sequence that is codon optimized for a host cell or is identical to a sequence as set forth in Genbank Accession Nos. BAB98576.1, CAF31258.1, AFD00624.1, or ADC46990.1, or comprises a consensus sequence of these homoserine dehydrogenases or comprises a consensus sequence of a plurality of known homoserine dehydrogenase polypeptides.

For example, to express or overproduce homoserine kinase, one or more genes from *E. coli* (*thrB*)*, Corynebacterium glutamicum* (*thrB*)*,* or *Methanococcus maripaludis* (*thrB*) can be introduced into and expressed or overexpressed in a hydrogenotrophic microorganism (*e.g*., methanogen), thereby producing or overproducing exogenous homoserine kinase or a functional fragment thereof. In certain embodiments, homoserine kinase polypeptides for use in the compositions and methods disclosed herein are from *Corynebacterium glutamicum* ATCC 13032 (Genbank Accession No. BAB98577.1), *Methanococcus maripaludis* S2 (Genbank Accession No. CAF29851.1), *E. coli* K-12 substr. W3110 thrB (GenBank Accession No. BAB96580.2), or *Methanocella conradii* HZ254 (Genbank Accession No. AFC99758.1).

In certain embodiments, a homoserine kinase amino acid sequence or a functional fragment thereof is based on the amino acid sequence of *E. coli* K-12 substr. W3110, *Corynebacterium glutamicum* ATCC 13032 or *Methanococcus maripaludis* S2 and is at least at least 75%, at least 80% identical, at least 85% identical, at least 90% identical, at least 91% identical, at least 92% identical, at least 93% identical, at least 94% identical, at least 95% identical, at least 96% identical, at least 97% identical, at least 98% identical, or at least 99% identical to the sequence set forth in Genbank Accession No. BAB96580.2, BAB98577.1 or CAF29851.1, respectively, or a functional fragment thereof. In other embodiments, a recombinantly encoded homoserine kinase has an amino acid sequence that is codon optimized for a host cell or is identical to a sequence as set forth in Genbank Accession Nos. BAB96580.2, BAB98577.1, CAF29851.1, or AFC99758.1, or comprises a consensus sequence of these homoserine kinases or comprises a consensus sequence of a plurality of known homoserine kinase polypeptides.

For example, to express or overproduce threonine synthase, one or more genes from *E. coli* (*thrC*)*, Corynebacterium glutamicum* (thrC), or *Methanococcus maripaludis* (thrC) can be introduced into and expressed or overexpressed in a hydrogenotrophic microorganism (*e.g*., methanogen), thereby producing or overproducing exogenous threonine synthase or a functional fragment thereof. In certain embodiments, threonine synthase polypeptides for use in the compositions and methods disclosed herein are from *Corynebacterium glutamicum* ATCC 13032 (Genbank Accession No. BAB99613.1), *Methanococcus maripaludis* S2 (Genbank Accession No. CAF29691.1), *Methanocella conradii* HZ254 (Genbank Accession No. AFD00584.1), *E. coli* K-12 substr. W3110 thrC (GenBank Accession No. BAB96581.1), or *Methanobrevibacter ruminantium* M1 (Genbank Accession No. ADC47342.1).

In certain embodiments, a threonine synthase amino acid sequence or a functional fragment thereof is based on the amino acid sequence of *E. coli* K-12 substr. W3110, *Corynebacterium glutamicum* ATCC 13032 or *Methanococcus maripaludis* S2 and is at least at least 75%, at least 80% identical, at least 85% identical, at least 90% identical, at least 91% identical, at least 92% identical, at least 93% identical, at least 94% identical, at least 95% identical, at least 96% identical, at least 97% identical, at least 98% identical, or at least 99% identical to the sequence set forth in Genbank Accession No. BAB96581.1, BAB99613.1 or CAF29691.1, respectively, or a functional fragment thereof. In other embodiments, a recombinantly encoded threonine synthase has an amino acid sequence that is codon optimized for a host cell or is identical to a sequence as set forth in Genbank Accession Nos. BAB96581.1, BAB99613.1, CAF29691.1, AFD00584.1, or ADC47342.1, or comprises a consensus sequence of these threonine synthases or comprises a consensus sequence of a plurality of known threonine synthase polypeptides.

In certain embodiments, a hydrogenotrophic microorganism may directly incorporate a sulfur source, such as H₂S, directly into the methionine biosynthetic pathway. Any sulfide that is produced or is present for use by a hydrogenotrophic microorganism can enter the homocysteine biosynthesis pathway wherein *O*-acetylhomoserine sulfhydrylase incorporates H₂S into *O*-acetylhomoserine to produce homocysteine, which can be further converted into methionine by methionine synthase (cobalamin dependent or independent).

For example, hydrogenotrophic microorganisms as described herein may be engineered to express or overproduce *O*-acetylhomoserine sulfhydrylase (EC 2.5.1.49), which can incorporate H₂S into *O*-acetyl-homoserine to produce homocysteine, and optionally engineered to express or overproduce cobalimin-dependent methionine synthase (EC 2.1.1.13) or cobalimin-independent methionine synthase (also known as homocysteine methyltransferase) (EC 2.1.1.14) to convert homocysteine into methionine.

To express or overproduce *O*-acetylhomoserine sulfhydrylase, one or more genes based on those from *Methanocella conradii* or *Methanobrevibacter ruminantium* can be introduced into and expressed or overexpressed in a hydrogenotrophic microorganism (*e.g*., non-natural or recombinant methanogen) of this disclosure, thereby producing or overproducing exogenous *O*-acetylhomoserine sulfhydrylase or a functional fragment thereof. In certain embodiments, *O*-acetylhomoserine sulfhydrylase polypeptides for use in the compositions and methods disclosed herein may be from *Methanocella conradii* HZ254 (Genbank Accession No. AFD00350.1), *Methanobrevibacter ruminantium* M1 (Genbank Accession No. ADC47419.1 or ADC46998.1), *Clostridium difficile* T19 (Genbank Accession No. ERM48664.1), *Clostridium botulinum* A str. ATCC 3502 (Genbank Accession No. CAL83417.1), *Leptospira meyeri* (Genbank Accession No. P94890.1), or *Rhodobacter sphaeroides* 2.4.1 (Genbank Accession No. YP351901.2).

In certain embodiments, an *O*-acetylhomoserine sulfhydrylase amino acid sequence or a functional fragment thereof is based on the amino acid sequence of *Methanocella conradii* HZ254 or *Methanobrevibacter ruminantium* M1 and is at least at least 75%, at least 80% identical, at least 85% identical, at least 90% identical, at least 91% identical, at least 92% identical, at least 93% identical, at least 94% identical, at least 95% identical, at least 96% identical, at least 97% identical, at least 98% identical, or at least 99% identical to the sequence set forth in Genbank Accession No. AFD00350.1 or ADC47419.1, respectively, or a functional fragment thereof. In other embodiments, a recombinantly encoded *O*-acetylhomoserine sulfhydrylase has an amino acid sequence that is codon optimized for a host cell or is identical to a sequence as set forth in Genbank Accession Nos. AFD00350.1, ADC47419.1, ADC46998.1, CCL83415.1, or CAL83417.1, or comprises a consensus sequence of these *O*-acetylhomoserine sulfhydrylases or comprises a consensus sequence of a plurality of known *O*-acetylhomoserine sulfhydrylase polypeptides.

In any of the aforementioned *O*-acetylhomoserine sulfhydrylase, a non-natural or recombinant hydrogenotrophic microorganism is further engineered to express, overexpress, or overproduce a homoserine *O*-acetyltransferase, as described herein.

In further embodiments, hydrogenotrophic microorganisms as described herein may be engineered to express or overproduce cobalamin-dependent methionine synthase (EC 2.1.1.13) or cobalamin-independent methionine synthase (also known as homocysteine methyltransferase) (EC 2.1.1.14), and optionally engineered to also express or overproduce *O*-acetylhomoserine sulfhydrylase.

For example, to express or overproduce cobalamin-dependent methionine synthase, one or more genes from *Escherichia coli* (metH), *Corynebacterium glutamicum* (metH), or *Clostridium difficile* can be introduced into and expressed or overexpressed in a hydrogenotrophic microorganism (*e.g*., non-natural methanotroph bacteria), thereby producing or overproducing exogenous cobalamin-dependent methionine synthase or a functional fragment thereof. In certain embodiments, cobalamin-dependent methionine synthase polypeptides for use in the compositions and methods disclosed herein are from *Escherichia coli* K-12 substrain MG1655 (Genbank Accession No. AAC76832.1), *Corynebacterium glutamicum* ATCC 13032 (Genbank Accession No. BAB98900.1), *Clostridium difficile* F665 (Genbank Accession No. ERM51559.1), or *Psuedomonas putida* GB-1 (Genbank Accession No. ABY97885.1).

In certain embodiments, a cobalamin-dependent methionine synthase amino acid sequence or a functional fragment thereof is based on the amino acid sequence of *Corynebacterium glutamicum* ATCC 13032 and is at least at least 75%, at least 80% identical, at least 85% identical, at least 90% identical, at least 91% identical, at least 92% identical, at least 93% identical, at least 94% identical, at least 95% identical, at least 96% identical, at least 97% identical, at least 98% identical, or at least 99% identical to the sequence set forth in Genbank Accession No. BAB98900.1, or a functional fragment thereof. In other embodiments, a recombinantly encoded cobalamin-dependent methionine synthase has an amino acid sequence that is codon optimized for a host cell or is identical to a sequence as set forth in Genbank Accession Nos. AAC76832.1, BAB98900.1, ERM51559.1, or ABY97885.1, or comprises a consensus sequence of these cobalamin-dependent methionine synthases or comprises a consensus sequence of a plurality of known cobalamin-dependent methionine synthase polypeptides.

In other embodiments, for example, to express or overproduce methionine synthase, one or more genes from *Escherichia coli* (metE or metB12), *Corynebacterium glutamicum* (metE), or *Methanococcus maripaludis* (metE) can be introduced into and expressed or overexpressed in a hydrogenotrophic microorganism (*e.g*., non-natural or recombinant methanogen), thereby producing or overproducing exogenous cobalamin-independent methionine synthase or a functional fragment thereof. In certain embodiments, cobalamin-independent methionine synthase polypeptides for use in the compositions and methods disclosed herein are from *Escherichia coli* K-12 substrain MG1655 (Genbank Accession No. AAC76832.1), *Corynebacterium glutamicum* ATCC 13032 (Genbank Accession No. CAF19845.1), *Methanococcus maripaludis* S2 (Genbank Accession No. NP_987521.1), *Methanocella conradii* HZ254 (Genbank Accession No. AFD00421.1), or *Methanobrevibacter ruminantium* M1 (Genbank Accession No. ADC47470.1).

In certain embodiments, a cobalamin-independent methionine synthase amino acid sequence or a functional fragment thereof is based on the amino acid sequence of *Methanococcus maripaludis* S2 or *Methanobrevibacter ruminantium* M1 and is at least at least 75%, at least 80% identical, at least 85% identical, at least 90% identical, at least 91% identical, at least 92% identical, at least 93% identical, at least 94% identical, at least 95% identical, at least 96% identical, at least 97% identical, at least 98% identical, or at least 99% identical to the sequence set forth in Genbank Accession No. NP_987521.1 or ADC47470.1, respectively, or a functional fragment thereof. In other embodiments, a recombinantly encoded cobalamin-independent methionine synthase has an amino acid sequence that is codon optimized for a host cell or is identical to a sequence as set forth in Genbank Accession Nos. AAC76832.1, CAF19845.1, NP_987521.1, AFD00421.1, or ADC47470.1, or comprises a consensus sequence of these cobalamin-independent methionine synthases or comprises a consensus sequence of a plurality of known cobalamin-independent methionine synthase polypeptides.

In any of the aforementioned methyl transferase embodiments, a non-natural hydrogenotrophic microorganism is further engineered to express, overexpress, or overproduce an *O*-acetylhomoserine sulfhydrylase, as described herein.

### H₂/COₓ Substrate

Hydrogen production involves a series of reforming, conditioning and separation steps wherein several of those steps (*e.g*., steam reforming, autothermal reforming, high temperature shift, low temperature shift, CO2 scrubbing and pressure swing absorption) can provide a feedstock that by itself or in combination with one or more other gas streams can provide an H₂/COₓ substrate useful as a feedstock for hydrogenotrophic microorganisms and methods of this disclosure.

By way of background, hydrogen production may involve single step or multistep reforming, partial oxidation or gasification to produce a H₂/COₓ substrate such as syngas, combined with a high temperature water gas shift (HTS) reaction, a low temperature water gas shift (LTS) reaction, or both. In some methods, carbon oxides are removed by using pressure swing adsorption (PSA) with molecular sieves, which separates a substantially pure hydrogen (H₂) gas stream from a tail gas comprising some residual H₂ gas along with various amounts of carbon dioxide (CO₂), carbon monoxide (CO), and methane (CH₄). In certain embodiments, carbon dioxide may be optionally scrubbed before subjecting the gas (*e.g*., syngas) to PSA. Depending on the syngas production process used and whether carbon dioxide is scrubbed, a tail gas will include different ratios of H₂, CO₂, CO, and CH₄. In some embodiments, a H₂/COₓ substrate for use in the methods of this disclosure is a gas stream blend comprising a mixture of PSA tail gas and H₂ gas.

For example, methane steam reforming combined with HTS will produce a gas stream having mostly H₂ (about 75%) and CO₂ (about 20%), with some CH₄ (about 5%) and very little or no CO. In another example, methane steam reforming combined with LTS will produce a gas stream having mostly H₂ (about 75%) and CO (about 10%), with some CO₂ (about 5%) and CH₄ (about 1%). In still another example, methane steam reforming combined with HTS and PSA will produce a tail gas having mostly H₂ (about 30%) and CO₂ (about 45%), with a fair amount of CO (about 10%) and CH₄ (about 15%). In this last embodiment, if a CO₂ scrubbing step is included, then the tail gas will comprise mostly H₂ (about 50%), CH₄ (about 30%) and CO (about 20%), with little CO₂ (about 1%). In certain embodiments, the PSA tail gas is mixed with the pipeline H₂ produced from PSA to produce a H₂/COₓ substrate of interest, such as a H₂/COₓ substrate having a H₂:CO₂ ratio of about 5:1, 4:1, 3:1, 2:1 or 1:1.

Steam reforming of methane can provide a feedstock ratio of CO₂ to H₂ that ranges from about 1:7 to about 1:15, respectively, wherein other components may include CO, CH₄ and H₂O. Alternatively, methane may be reformed with CO₂, which is called dry reforming. Dry reforming of methane can provide a feedstock ratio of CO₂ to H₂ that ranges from about 1:5 to about 1:15, respectively, wherein other components may include CO, CH₄, and H₂O.

Partial oxidation (catalytic or non-catalytic) and autothermal reforming use oxygen as a co-reactant to natural gas instead of water. Partial oxidation and autothermal reforming can provide a feedstock ratio of CO₂ to H₂ that is about 1:20, wherein other components may include CO, CH₄, and H₂O.

Gasification, the partial oxidation of carbon containing material with air or oxygen (*e.g*., natural gas liquids, naphtha, bitumen, coal, biomass, or the like), can provide a H₂/COₓ feedstock for use with the hydrogenotrophic microorganisms and methods of this disclosure. For example, the gasification of coal provides a feedstock ratio of CO₂ to H₂ that ranges from about 1:1.1 to about 1:11, respectively, wherein other components may include CO, CH₄, N₂, and H₂O.

Ammonia synthesis involves series of reforming and conditioning steps, wherein four (steam reforming, autothermal reforming, high temperature shift, low temperature shift) of those steps can provide a H₂/COₓ feedstock for use with the hydrogenotrophic microorganisms and methods of this disclosure. For each of these different processes, a feedstock ratio of CO₂ to H₂ that ranges from about 1:3 to about 1:10, respectively, is provided, wherein other components may include CO, CH₄, N₂, and H₂O.

Methanol synthesis involves the steps of low temperature reforming, steam reforming, and autothermal reforming, all of which can provide a H₂/COₓ feedstock for use with the hydrogenotrophic microorganisms and methods of this disclosure. For each of these three processes, a feedstock ratio of CO₂ to H₂ that ranges from about 1:7 to about 1:12, respectively, is produced, wherein other components may include CO, CH₄, and H₂O.

An integrated steel mill combines various processes, including a coke oven (to make coke from coal), a blast furnace (to make pig iron) and an oxygen furnace (to make steel). In certain embodiments, direct reduction in an integrated steel mill uses reformed natural gas as a reductant (instead of coke) to make pig iron. Each of these ovens, as well as direct reduction reforming (which produces top gas), can produce a feedstock ratio of CO₂ to H₂ that ranges from about 8:1 (from blast or oxygen furnace) to about 1:32 (from coke oven), respectively, wherein other components may include CO, CH₄, C₂H₆, C₃H₈, N₂, and H₂O.

In any of the aforementioned sources of H₂/COₓ substrate, a H₂/COₓ feedstock so produced can be mixed any other produced H₂/COₓ feedstock or with H₂, CO₂, CO or any combination thereof to produce a H₂/COₓ substrate of interest, such as a substrate having a H₂:CO₂ ratio of about 4:1 or 3:1. In certain embodiments, a H₂/COₓ substrate for use with, for example, methanogens, comprises a H₂:CO₂ ratio of about 5:1, 4:1, 3:1, 2:1 or 1:1, and optionally the total amount of CO is no more than about 0.1%, 0.2%, 0.3%, 0.4%, 0.5%, 0.6%, 0.7%, 0.9%, 0.9%, 1.0%, 2.0%, 3.0%, 4.0%, 5.0%, 6.0%, 7.0%, 8.0%, 9.0%, 10%, 11%, 12%, 13%, 14%, 15%, or 20%. In other embodiments, a H₂/COₓ substrate for use with, for example, *Clostridium,* comprises a H₂:(CO₂ + CO) ratio of about 5:1, 4:1, 3:1, 2:1, or 1:1, and optionally the total amount of CO is at least about 1.0%, 5.0%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, or more. In any of these embodiments, the H₂/COₓ substrate may comprise a blend of PSA tail gas with H₂ gas.

In any of the aforementioned non-natural hydrogenotrophic microorganism embodiments, the present disclosure provides hydrogenotrophic microorganisms that utilize, metabolize, oxidize, or convert a H₂/COₓ substrate comprised of H₂ with CO₂ or CO or both, and optionally various other components as described herein. In certain embodiments, a H₂/COₓ substrate is H₂, CO₂ and CO, wherein there is more CO than CO₂, optionally with various other components as described herein. In further embodiments, a H₂/COₓ substrate is H₂, CO₂ and CO, wherein there is more CO₂ than CO, optionally with various other components as described herein. In yet further embodiments, a H₂/COₓ substrate is H₂, CO₂ and CO wherein there is more CO than both CO₂ and H₂, optionally with various other components as described herein. In certain circumstances, a microorganism that metabolizes H₂/COₓ may use the H₂ as an energy source and the COₓ, wherein x is 1 or 2, as a carbon source, or use H₂ and COₓ as an energy source, and COₓ as a carbon source.

In any of the aforementioned non-natural hydrogenotrophic microorganism embodiments, the present disclosure provides a H₂/COₓ substrate that can be produced, for example, by steam reforming, dry reforming, autothermal reforming, catalytic partial oxidation or partial oxidation of natural gas or liquid hydrocarbons (*e.g*., ethane, propane, naphtha), within hydrogen production, within ammonia synthesis, within methanol synthesis, by steelmaking, or by gasification of coal, naphtha, resid, biomass or waste. In certain embodiments, a H₂/COₓ substrate produced by any of the aforementioned reforming methods can be further conditioned by a water-gas shift reaction. In addition, one or more gas streams produced by any of the aforementioned methods can be blended with other sources of hydrogen, carbon monoxide or carbon dioxide to produce or make a H₂/COₓ substrate, including pipeline hydrogen, pipeline carbon dioxide, carbon dioxide scrubber off-gas, flue gas, ethane cracker off-gas, reformer off-gas or chlorine synthesis off-gas. In some embodiments, the feedstock ratio of CO₂ to H₂ ranges from about 1:50 to about 10:1, respectively. In further embodiments, the feedstock ratio of CO₂ to H₂ ranges from about 1:3 to about 1:5, respectively.

### Culture Methods

A variety of culture methodologies may be used for non-natural hydrogenotrophic microorganisms (*e.g*., bacteria, methanogenic archaea) described herein. For example, hydrogenotrophic microorganisms may be grown by batch culture or continuous culture methodologies. In certain embodiments, cultures are grown in a controlled culture unit, such as a fermenter, bioreactor, hollow fiber membrane bioreactor, bubble column bioreactor, trickle bed bioreactor, or the like.

A classical batch culturing method is a closed system where the composition of the media is set at the beginning of the culture and not subject to external alterations during the culture process. Thus, at the beginning of the culturing process, the media is inoculated with the desired hydrogenotrophic microorganism (*e.g*., methanogen) and growth or metabolic activity is permitted to occur without adding anything to the system. Generally, a "batch" culture is batch with respect to the addition of carbon source, gas feedstock and media components, wherein waste gasses are allowed to exit, and attempts are often made at controlling other factors, such as pH. In batch systems, the metabolite and biomass compositions of the system change constantly up to the time the culture is terminated. Within batch cultures, cells moderate through a static lag phase to a high growth logarithmic phase and finally to a stationary phase where growth rate is diminished or halted. If untreated, cells in the stationary phase will eventually die. Cells in logarithmic growth phase are often responsible for the bulk production of end product or intermediate in some systems. Stationary or post-exponential phase production can be obtained in other systems.

A Fed-Batch system is a variation on the standard batch system. Fed-Batch culture processes comprise a batch system with the modification that a substrate and potentially media components are added in increments as the culture progresses. Fed-Batch systems are useful when catabolite repression is apt to inhibit the metabolism of the cells and where it is desirable to have limited amounts of substrate in the media. In gas substrate fermentations, a system is continuous with respect to gas substrate (since waste gas can be removed) and Fed-batch with respect to liquid (media). Batch and Fed-Batch culturing methods are common and known in the art (*see, e.g.,* Thomas D. Brock, Biotechnology: A Textbook of Industrial Microbiology, 2nd Ed. (1989) Sinauer Associates, Inc., Sunderland, MA; Deshpande, Appl. Biochem. Biotechnol. 36:227, 1992).

Continuous cultures are "open" systems where a defined culture media is added continuously to a bioreactor and an equal amount of conditioned media is removed simultaneously (with or without biomass or cell retention) for processing. Continuous cultures generally maintain the cells at a constant high liquid phase density where cells are primarily in logarithmic phase growth. Alternatively, continuous culture may involve biomass, cell retention or cell immobilization where feedstock and nutrients are continuously added and valuable products, by-products, and waste products can be continuously removed from the cell mass. Cell retention may be performed by a variety of methods, such as by filtration, centrifugation or settling. Cell immobilization may be performed using a wide range of solid supports composed of natural and/or synthetic materials.

Continuous or semi-continuous culture allows for the modulation of one factor or any number of factors that affect cell growth or end product concentration. For example, one method can maintain a limited nutrient (*e.g*., carbon source, nitrogen level, hydrogen level, phosphorous level) at a fixed rate and allow all other parameters to modulate. In other systems, a number of factors affecting growth can be altered continuously while the cell concentration, measured by media turbidity, is kept constant. In certain embodiments, hydrogenotrophic biomass growth is limited to increase product to biomass ratio. Methods of modulating nutrients and growth factors for continuous culture processes, as well as techniques for maximizing the rate of product formation, are well known in the art (*see* Brock, 1989).

Liquid phase bioreactors (*e.g*., stirred tank, packed bed, one liquid phase, two liquid phase, hollow fiber membrane) are well known in the art and may be used for growth of hydrogenotrophic microorganisms.

Multiphase bioreactors may be used in the methods of the instant disclosure (*e.g*., bubble column reactor, trickle bed reactor (fixed or packed bed), fluidized bed reactor). Bubble columns are the devices in which gas, in the form of bubbles, come in contact with the liquid. Trickle bed reactors use co-current or countercurrent flow of gas and liquid to grow cultures. A fluidized bed reactor comprises passing a fluid (gas or liquid) through a granular solid material at high enough velocities to suspend the solid and cause it to behave as though it were a fluid. One purpose of multiphase bioreactors is to mix the liquid and gas phases, wherein the gas is consumed by hydrogenotrophic microorganisms to a greater or lesser extent depending on the intensity of mass transfer and chemical reaction. Various types of multiphase bioreactors are well known in the art and may be used for growth of hydrogenotrophic microorganisms in the methods of the instant disclosure.

Hydrogenotrophic microorganisms described in the present disclosure may be grown as an isolated pure culture, with a heterologous non-hydrogenotrophic microorganism(s) that may aid with growth, or combined with one or more different strains or species of hydrogenotrophic microorganisms to generate a mixed culture.

In other aspects, this disclosure provides a method for producing an aspartate pathway amino acid or an aspartate pathway amino acid-containing feed additive, comprising culturing any of the aforementioned non-natural or recombinant hydrogenotrophic microorganisms for a time sufficient to produce one or more aspartate pathway amino acids, wherein the non-natural or recombinant hydrogenotrophic microorganism: (a) expresses one or more aspartate pathway enzymes having increased activity as compared to a parent hydrogenotrophic microorganism; (b) overexpresses one or more aspartate pathway enzymes; or (c) comprises altered regulation of one or more aspartate pathway enzymes, wherein the non-natural hydrogenotrophic microorganism produces one or more aspartate pathway amino acids at a higher level than a parent hydrogenotrophic microorganism.

In certain embodiments, the present disclosure provides a process for making an aspartate pathway amino acid or an aspartate pathway amino acid-containing feed additive, comprising culturing a recombinant, aspartate pathway amino acid-excreting hydrogenotrophic microorganism of this disclosure in the presence of a H₂/COₓ substrate under conditions and for a time sufficient to allow for expression of an exogenous polynucleotide encoding a polypeptide from one or more pathways for biosynthesis of an aspartate family amino acid, wherein one or more aspartate pathway amino acids are produced and accumulate in the culture medium at a higher level than the one or more aspartate pathway amino acids produced by a parent hydrogenotrophic microorganism.

In any of the aforementioned methods, the hydrogenotrophic microorganisms can be cultured in a fermenter or bioreactor, such as a liquid phase, bubble column, or trickle bed bioreactor.

In any of the aforementioned methods for using non-natural hydrogenotrophic microorganisms (*e.g*., methanogen) to produce aspartate pathway amino acids as disclosed herein, the gas feedstock is a H₂/COₓ substrate, wherein the feedstock comprises H₂ with CO₂ or CO or both, and optionally various other components as described herein. In certain embodiments, a H₂/COₓ substrate is syngas, such as syngas produced by steam reforming, dry reforming, autothermal reforming, catalytic partial oxidation or partial oxidation of natural gas or liquid hydrocarbons, conditioned by a water-gas shift reaction, by ammonia synthesis, by methanol synthesis, by steelmaking, or by gasification of coal, biomass or waste.

In any of the aforementioned methods for using non-natural hydrogenotrophic microorganisms (*e.g*., methanogen) to produce aspartate pathway amino acids as disclosed herein, a gas substrate is a H₂/COₓ substrate, which can be produced, for example, by steam reforming, dry reforming, autothermal reforming, catalytic partial oxidation or partial oxidation of natural gas or light hydrocarbons (*e.g*., ethane, propane, naphtha), conditioned by a water-gas shift reaction, within hydrogen production, within ammonia synthesis, within methanol synthesis, by steelmaking, or by gasification of coal, naphtha, resid, biomass or waste. In certain embodiments, a H₂/COₓ substrate is a blend of any gas stream so produced with one or more other sources of hydrogen, carbon monoxide, carbon dioxide or any combination thereof, including pipeline hydrogen, pipeline carbon dioxide, carbon dioxide scrubber off-gas, flue gas, ethane cracker off-gas, reformer off-gas chlorine synthesis off-gas, or any combination thereof.

In certain embodiments, methods for converting a H₂/COₓ substrate into an aspartate pathway amino acid of interest as provided herein will produce at least about or up to 1 kilogram (kg), at least about or up to 10 kg, at least about or up to 100 kg, at least about or up to 1,000 kg, at least about or up to 10,000 kg, at least about or up to 50,000 kg, at least about or up to 100,000 kg, at least about or up to 250,000 kg, at least about or up to 500,000 kg, or more of an amino acid of interest/day. In certain embodiments, one or more aspartate pathway amino acids are produced at about 100,000 metric tons (MT) per year (*i.e.,* 100 million kg per year or 300,000 kg/day), about 75,000 MT per year (or 225,000 kg/day), about 50,000 MT per year (or 150,000 kg/day), about 25,000 MT (or 75,000 kg/day), or about 10,000 MT per year (or 30,000 kg/day).

### Systems for Making Amino Acids

In additional aspects, the present disclosure provides a system for producing an aspartate pathway amino acid, comprising a source of gas comprising a H₂/COₓ substrate; a bioreactor comprising any one or more of the aforementioned non-natural hydrogenotrophic microorganisms that (a) expresses one or more aspartate pathway enzymes having increased activity as compared to a parent hydrogenotrophic microorganism; (b) overexpresses one or more aspartate pathway enzymes; or (c) comprises altered regulation of one or more aspartate pathway enzymes,; and a connector disposed between the gas source and the bioreactor to allow flow of the gas into the bioreactor; wherein the non-natural hydrogenotrophic microorganism metabolizes the H₂/COₓ substrate to overproduce one or more aspartate pathway amino acids as compared to a parent hydrogenotrophic microorganism.

In any of the aforementioned systems, the H₂/COₓ substrate is converted into a biological material, such as animal feed or a fertilizer. In certain embodiments, the H₂/COₓ substrate is assimilated into a biological material enriched for one or more aspartate family amino acids, such as lysine, threonine, methionine, or any combination thereof. In further embodiments, the resultant one or more aspartate family amino acids are purified and used as animal feed, food additives or nutrient supplements. In still other embodiments, biomass enriched with one or more aspartate family amino acids are used for animal feed, food additives or nutrient supplements.

In any of the aforementioned systems for using non-natural hydrogenotrophic microorganisms (*e.g*., methanogen) to produce aspartate pathway amino acids as disclosed herein, the gas feedstock is a H₂/COₓ substrate, wherein the feedstock comprises H₂ with CO₂ or CO or both, and optionally various other components as described herein. In certain embodiments, a H₂/COₓ substrate is syngas, such as syngas produced by steam reforming, dry reforming, autothermal reforming, catalytic partial oxidation or partial oxidation of natural gas or light hydrocarbons (*e.g*., ethane, propane, naphtha), conditioned by a water-gas shift reaction, within ammonia synthesis, within methanol synthesis, by steelmaking, or by gasification of coal, naphtha, resid, biomass or waste. In certain embodiments, a H₂/COₓ substrate is a blend of any gas stream so produced with one or more other sources of hydrogen, carbon monoxide, carbon dioxide or any combination thereof, including pipeline hydrogen, pipeline carbon dioxide, carbon dioxide scrubber off-gas, flue gas, ethane cracker off-gas, reformer off-gas chlorine synthesis off-gas, or any combination thereof.

### EXAMPLES

### EXAMPLE 1

### LYSC MUTANTS OF HYDROGENOTROPHIC MICROORGANISMS

A first step is to isolate feedback resistant lysC (aspartokinase) mutants of *M. maripaludis.* For example, one or more specific mutations are engineered into the aspartokinase gene of *M. maripaludis* or spontaneous aspartokinase mutants are identified by exposing a wild-type or parent *M. maripaludis* to a toxic lysine analog, such as S-2-aminoethyl-L-cysteine (AEC), and identifying *M. maripaludis* capable of growing in the presence of (resistant to) AEC. The resistance to a toxic analog may be spontaneous, or may be induced using a mutagen, such as EMS. The toxic analog may be combined with other amino acid analogs, such as methionine or threonine, or may be used alone, to select for *M. maripaludis* mutants resistant to the toxic analog. Additionally, aspartokinase having feedback resistance (deregulated) is selected by identifying mutants that are no longer susceptible to growth inhibition in the presence of regulatory agents, such as threonine, lysine or a combination of the two. Again this feedback resistance may be spontaneous, induced by a chemical mutagen, or site-specifically introduced.

Briefly, wild-type *Methanococcus maripaludis* Trel10 was grown in 25 mL McCas media without Casamino acids (for media recipe, *see* Sarmiento et al., Methods Enzymol. 494:44, 2011; which refers to the media McCV, and here is used without Casamino acids or yeast extract) at 37°C to an OD₆₀₀ of approximately 0.20. Five mL of culture was dispensed into two anaerobic Balch tubes. To one tube, 0.3 mL ethyl methanesulfonate (EMS, 1:50 dilution in McCas no Cas) was added, and to the second tube, 0.3mL of McCas no cas was added (control). Tubes were pressurized with an 80/20 mix of H₂/CO₂ to 40 PSIG and incubated without shaking at 37°C for one hour. After one hour incubation, pressure was released and 0.5mL of each tube was removed and plated on McCas agar plates to determine the kill rate.

The remainder of the cultures was dispensed into 5 (EMS treated) or 2 (Control), 1.5 sterile anaerobic microcentrifuge tubes and centrifuged at 1000g for 15 minutes. The supernatant was removed, the cell pellet washed by resuspending in 1mL McCas no Cas, and centrifuging again at 1000g for 15 minutes. This wash was repeated 2x to remove all the traces of EMS. After the final wash, the harvested cells were suspended in 200 µL McCas no Cas and transferred to 5mL McCas no Cas for recovery. 1 mL 2.5% Na₂S x 9H₂0 was added to each tube and each tube was pressurized to 40 PSIG with 80/20 H₂/CO₂ and allowed to recover overnight at 37°C. The next morning, each culture was concentrated to 0.1mL by centrifugation and plated on McCas no Cas plates containing 0.1M threonine and 0.02M AEC. Plates were incubated in an Oxoid anaerobic jar with 10PSIG of an 80/20 H₂/CO₂ gas mix at 37°C. The selection was such that colonies grew only on plates inoculated with EMS treated cells. All work was carried out under anaerobic conditions unless otherwise stated.

Figure 1 shows that the growth rate of EMS generated *lysC* mutant Trel10-Mut333, which has a G333R mutation (corresponding to LysC amino acid position G277 of *Corynebacterium glutamicum* ATCC 13032), is not affected when grown in presence of lysine and threonine (OD₆₀₀ was measured after 72 hours of incubation at 37°C) - in other words, the mutated aspartokinase of Trel10-Mut333 is not subject to feedback inhibition by lysine and threonine. Figure 2 shows the aspartate pathway amino acids produced by the Trel10-Mut333 mutant and identified by HPLC. The derivatizing agent ortho-phthalaldehyde (OPA) was used in an automated derivatization reaction on an autosampler and was done pre-column. The reaction mixture was buffered at pH 10.2 (via Borate Buffer), which allowed direct derivatization of acid hydrolyzed protein/peptide samples. The amino acids of interest were reacted first with OPA using 3-mercaptopropionic acid (3-MPA). The incorporation of the 3-MPA into the indoles decreases their hydrophobicity, and as a result, the OPA-derivatives eluted chromatographically. The production profile of the Trel10-Mut333 mutant as compared to the parent strain was as follows (and generally similar in all mutants identified, data not shown): alanine (5 mg/L), lysine (8 mg/L), threonine (21 mg/L), and glycine (78 mg/L).

Mutations in the *M. maripaludis lysC* were verified by extracting genomic DNA using the Qiagen DNAeasy Blood & Tissue Kit following the protocol for gram negative bacteria. LysC targets were amplified using the Easy-A high fidelity polymerase with forward primer (LysCfor1 - 5'GGGACGGCGCAACAAATGG3'; SEQ ID NO.:1) and reverse primer (LysCrev1 - 5'GGAGATAGTGAGACCCCTGGAGT3'; SEQ ID NO.:2). Amplified DNA was mixed with either LysCfor1 or LysCrev1 and sequenced (Operon). One spontaneous mutant and 8 chemically induced mutants were identified. In addition to mutation at postion G277 previously identified in *Corynebacterium* (in this case, G277R), new mutation positions not previously identified in *Corynebacterium* were found, including S302P and G359E (numbering according to amino acid positions from LysC of *Corynebacterium glutamicum* ATCC 13032).

A similar approach was taken with *Methanococcus vannielii,* wherein a colony isolate of *Methanococcus vannielli* SB (DSMZ 1224) was used and referred to as Trel15. The procedure *Methanococcus vannielii* was similar to that undertakedn with *M. maripaludis* except that selection was with 0.02M AEC without threonine. Liquid chromatography (LC) results showed an increase in lysine production in AEC resistant mutants. Sequencing showed two Trel15 mutants had mutations in the putative LysC protein. In particular, at positions F339G and K380Q (amino acid positions corresponding to *M. vannielii* protein).

Trel15 LC data showed an amino acid profile of 0 (not detectable) mg/L Lysine, 50 mg/L glycine, 2.5 mg/L threonine, while Trel15-Mut 339 had an amino acid profile of 8.6 mg/L Lysine, 37.5mg/L glycine and 6.1 mg/L threonine, and Trel15-Mut380 had an amino acid profile of 4.3 mg/L Lysine, 41.5 mg/L Glycine, and 6.0 mg/L threonine.

### EXAMPLE 2

### AMINO ACID OVERPRODUCING HYDROGENOTROPHIC MICROORGANISMS

An initial step is to identify *lysC* mutants as described in Example 1. A second step to overproduce lysine is to inactivate pathways downstream of the lysine branch. For example, homoserine dehydrogenase, or any gene downstream of homoserine dehydrogenase, is inactivated, which can include genes for threonine biosynthesis and/or methionine biosynthesis. Inactivation or reduced activity is done by gene deletion, or by selecting for homoserine, threonine, and/or methionine auxotrophs by spontaneous mutation or chemically induced mutation, as described herein.

Optionally, any gene of the lysine biosynthetic pathway is overexpressed, including lysC, deregulated lysC, asd, dapA, dapB, dapL, lysA or any combination thereof. For example, a native promoter is replaced with one or more stronger promoters or by supplying additional copies of the gene expressed by its native promoter, or both. For example, the histone-like promoter (hmv) from *Methanococcus voltae* was operably linked to various genes of interest, including *dapA, dapAB* or *dapABL* genes, to create a multipcopy plasmid used to transform *M. maripaludis* strains as described herein. One such strain, Trel10-Mut333, has a mutated *lysC* gene on the chromosome which encodes a feedback resistant aspartokinase. Table 2 shows the aspartate amino acids produced by a *lysC* mutant overespressing DapA, DapAB, or DapABL. Exemplary modified *Methanococcus maripaludis* are provided in Table 1.

**Table 1. Modified Methanococcus maripaludis S2**

| **Strain Name** | **Relative Phenotype / Genotype** |
|---|---|
| Trel10 | Wild-type *Methanococcus maripaludis* S2 |
| Trel10-tA | thrA from *E. coli* transformed into Trel10 |
| Trel10-Mut333 | Trel10 with feedback resistant aspartokinase |
| Trel10-333dA | Trel10-Mut333 with plasmid expressing DapA from Trel10 |
| Trel10-333dAB | Trel10-333 with plasmid expressing DapAB from Trel 10 |
| Trel10-333UR | Trel10-333UR with *upp*:*repA*; has approximately the same amino acid production as Trel10-Mut333; change facilitates genetic engineering |
| Trel10-333UR-tABC | Trel10-333UR with plasmid containing thrABC from E. coli |
| Trel10-333UR-AtABC | Trel10-333UR with plasmid containing thrABC from *E*. *coli,* and *asd from M. maripaludis.* |
| Trel10-333dABL | Trel10-333UR with plasmid containing DapABL |
| Trel10-333UR-ΔdA | Trel10-333UR with a dapA deletion; requires lysine for growth |

In addition, exogenous genes encoding enzymes for alternate lysine biosynthetic pathways are introduced into a hydrogenotrophic microorganism (*e.g., M. maripaludis*)*.* For example, a ddh pathway for lysine biosynthesis is inserted into a hydrogenotrophic microorganism that does not usually have or use this pathway. Transformation of *Methanococcus maripaludis* is carried out as described by Sarmiento *et al.,* 2011. Plasmid DNA is purified using Qiagen plasmid DNA extraction kits as described by the manufacturer.

### EXAMPLE 3

### URACIL PHOSPHORIBOSYLTRANSFERASE DELETION AND REPA INSERTION IN HYDROGENOTROPHIC MICROORGANISMS

In order to improve plasmid transformation efficiency, *lysC* mutant *Methanococcus maripaludis* Trel10-Mut333 was modified on the genomic level by replacing the uracil phosphoribosyltransferase (*upp*) gene (Locus MMP0680) with the gene encoding replication protein A (*repA,* with its own promoter), referred to as Trel10-333UR. The *repA* allows for efficient transformation of any plasmid having *repA,* such as a plasmid derived from or based on the *repA*-containing pURB500 plasmid (*see* Tumbula et al., J. Bacteriol. 179:2976, 1997). The loss of uracil phosphoriboxyltransferase activity gives the modified *M. maripaludis* a 6-azaurcil resistance phenotype.

Briefly, the *repA* gene was amplified (along with its promoter) from the genomic DNA of *Methanococcus maripaludis* S001 (Walters et al., App. Environ. Microbiol. 77:2549, 2011) with primers TKH_038 (5'aaattatgaggcgcgcctccctgaagaagaagagag3', SEQ ID NO.:3) and TKH_039 (5'tgcttattcggcgcgccagttccattttaccacc3', SEQ ID NO.:4). The amplified *repA* fragment was cloned using the In-fusion® HD cloning kit (Clontech) into pCR® 2.1-TOPO® TA vector linearized with *Asc*I*.* The final plasmid was named pKH11. The *Xba*I*-BamH*I fragment from pKH11 was cloned into pMEV1 (Gardner WL (2000) Expression vectors for the methane-producing archaeon *Methanococcus maripaludis.* Dissertation, University of Georgia) linearized with restriction enzymes *Nhe*I and *Bgl*II*.* The resultant suicide vector carrying a puromycin resistance gene was named pKH20.

Plasmid pKH20 was transformed into Trel10-Mut333 essentially as described by Sarmiento *et al.* (2011), and transformants were selected on McCAS plates containing puromycin (2.5 mg/L). Transformant colonies that grew in presence of puromycin were transferred into McCAS liquid medium supplemented with 6-azauracil (0.25 mg/ml) and grown overnight. A portion of the overnight culture was transferred into fresh McCAS medium supplemented with 0.5 mg/ml 6-azauracil and grown overnight again. The cultures were diluted and then spread onto McCAS plates containing 0.25 mg/ml 6-azauracil. After 5 days, individual colonies were replica plated onto McCAS plates with or without puromycin. Colonies that failed to grow in presence of puromycin were transferred into McCAS liquid medium supplemented with 6-azauracil (0.25 mg/ml) to confirm resistance. The replacement of the *upp* gene with the *repA* gene on the Trel10-Mut333 genome was verified by PCR using the following primers: uptdelconf1 (5'-caattactgaacccaaagaccat-3', SEQ ID NO.:5) and uptdelconf2 (5'-aatagttaccggcgttacaatca-3', SEQ ID NO.:6). The 6-azauracil resistant / puromycin sensitive colony with the verified *upp* gene replacement with the *repA* gene was named Trel10-333UR.

### EXAMPLE 4

### DAPA DELETION IN HYDROGENOTROPHIC MICROORGANISMS

Construction of Trel10-333UR-ΔdA - a *dapA* deletion for increased methionine andtThreonine production was generated using essentially the same markerless mutagenesis method described in Sarmiento *et al.* (2011). An approximately 2.4 kb fragment from the *M. maripaludis* S2 (Trel10) genome containing the *dapA* gene, along with upstream and downstream regions, was synthesized via PCR using primers DapAfor2 (5'-tccctgatcgatagaaagtgtagt-3') (SEQ ID NO: 12) and DapArev2 (5'-ttgccgatgaaattaaagtgaaa-3') (SEQ ID NO: 13) and cloned into plasmid pTOPO to create pJB012. An in-frame deletion fragment of the dapA gene was created by using outward PCR with primers DapAdelfor2 (5'-gcgggcgcgccgcataattacaccttatgcgttc-3', SEQ ID NO.:14) and DapAdelrev2 (5'-gcgggcgcgcctaatcacggttcgtgatactat-3', SEQ ID NO.:15) both of which contain a 5' *Asc*I site. The PCR products were purified, digested with *Asc*I and ligated into pTOPO to create pJB013. Finally, a fragment containing the upp::neo gene was PCR amplified using primers uppneoF (5'-attacgccaagcttggtaccactctcttcttcttcaggga-3', SEQ ID NO.:16) and uppneoR (5'-gtggatccgagctcggtacctgagatccccgcgctggagg-3', SEQ ID NO.:17) from pKH14 and cloned into the *Kpn*I site of pJB013 to create pJB015.

pJB015 was transformed into Trel10-333UR (as described previously) selecting for neomycin (500ug mg/ml) resistant colonies on agar plates. A single crossover event at a chromosomal *dapA* gene was confirmed by PCR. Colonies with a single crossover were grown in non-selective media for 24 hours to allow a double crossover event, and then plated on McC media containing 100mg/L lysine and 0.25ug/ml 6-azauracil. Colonies were patched to McC plates with or without lysine. PCR confirmation of the *dapA* deletion was performed on colonies requiring lysine for growth. One such colony was designated Trel10-333UR-ΔdA.

### EXAMPLE 5

### ENHANCING GYCINE AND THREONINE PRODUCTION IN HYDROGENOTROPHIC MICROORGANISMS

Enhancement of glycine and threonine production was accomplished by increasing flux from OAA to threonine. Plasmid pAW42 (described by Walters *et al.,* 2011) was used as a replication plasmid for *Methanococcus maripadulis* (acquired from Professor Chong of University of York, Wentworth, York YO10 5 DD, United Kingdom).

### pKH24 (pAW42-thrA*) construction

The thrA* gene, encoding a dual function enzyme aspartate kinase/homoserine dehydrogenase that is feedback resistant to threonine (amino acid mutation G433R), was amplified from *E. coli* ATCC 21277 genome with primers TKH_094 (5'aactaataggtgaaacgcgtacaggaaacacagaaaaaagcc3', SEQ ID NO.:7) and TKH_095 (5'gatctcctaggcgcgcctcagactcctaacttccatgagagggtac3', SEQ ID NO.:8) and cloned into pAW42 linearized with *Asc*I and *Nsi*I*.* The resultant plasmid was named pKH24.

### pKH27 (pAW42-thrA*BC) construction

A gene cluster encoding a threonine feedback resistant aspartokinase/homoserine dehydrogenase (ThrA*), a homoserine kinase (ThrB), and a threonine synthase (ThrC) was amplified from the genomic DNA of *E. coli* ATCC 21277 with primers TKH_094 (SEQ ID NO.:7) and TKH_103 (5'gatctcctaggcgcgccttactgatgattcatcatcaatttacgcaacgca3', SEQ ID NO.:9). The PCR product was cloned into pAW42 linearized with *Nsi*I and *Ase*I. The resultant plasmid was named pKH27.

### pKH32 (pAW42-thrA*BC-asd) construction

The asd gene of *Methanococcus maripaludis* encodes aspartate semialdehyde dehydrogenase. The gene and its upstream sequences (including RBS) were amplified from the host genome with primers TKH115 (5'gatctcctaggcgcgccttaaaggtatttttgaacgaataattcagc3', SEQ ID NO.:10) and TKH116 (5'catcagtaaggcgcg tttttatccaaaggtgaaagaatgaa3', SEQ ID NO.:11). The resultant PCR product was cloned into pKH27 linearized with *Asc*I to produce pKH32.

### Fermentation Protocol for Amino Acid Analysis

*Methanococcus maripaludis* recombinants were grown in 5 ml of McCAS medium in a balch tube, supplemented with puromycin (2.5 mg/L), gased with H₂:CO₂ (4:1) to 40 psi at 37°C with shaking overnight. On the second day, 100 µl of the overnight culture was used as seed culture to inoculate 5 ml minimal media (MM) in a balch tube or 100 ml serum medium, gassed with H₂:CO₂ (4:1) to a pressure of 40 and 20 psi, respectively. The culture was placed at 37°C with shaking for 72 hours; H₂:CO₂ (4:1) gas was refilled to the full pressure at the beginning of culture. After fermentation, 1.8 ml of culture was transferred to an Eppendorf tube, cells were removed at 12000xg and the resultant supernatant was passed through 0.2 µm filter. The filtrate was analyzed by HPLC for amino acid content. If needed, both the seed and fermentation media were supplemented with puromycin (2.5 mg/L).

**Table 2. Amino Acid Production in Serum Tubes**

| **Strain** | **Amino Acid (mg/L)** | | | |
|---|---|---|---|---|
| | **Glycine** | **Threonine** | **Lysine** | **Methionine** |
| Trel 10 | 7 | 31 | ND | ND |
| Trel10-tA | 111.4 | 33.6 | 9.3 | ND |
| Trel10-Mut333 | 208 | 24 | 22 | ND |
| Trel10-333dA | 43 | 45 | 18 | ND |
| Trel10-333dAB | 86 | 31 | 41 | ND |
| Trel10-333UR | 149 | 10 | 12 | ND |
| Trel10-333UR-tABC | 178 | 10 | ND | ND |
| Trel10-333UR-AtABC | 197 | 10 | ND | ND |
| Trel10-333dABL | 227 | 57 | 118 | ND |
| Trel10-333UR-ΔdA | 166 | 17 | NA | 3 |

In addition, non-natural and recombinant *Methanococcus maripaludis* were grown in a bioreactor as described in Example 9, but without the addition of CO. After fermentation for 96 hours, 1.8 ml of culture was transferred to an Eppendorf tube, cells were removed at 12000xg and the resultant supernatant was passed through 0.2 µm filter. The filtrate was analyzed by HPLC for amino acid content. If needed, both the seed and fermentation media were supplemented with puromycin (2.5 mg/L).

**Table 3. Amino Acid Production in Bioreactor**

| **Strain** | **Amino Acid (mg/L)** | | | |
|---|---|---|---|---|
| | **Glycine** | **Threonine** | **Lysine** | **Total** |
| Trel 10 | 0 | 6 | 0 | 6 |
| Trel10-Mut333 | 233 | 30 | 32 | 295 |
| Trel10-333UR-AtABC | 1084 | 845 | 5 | 1934 |

As is evident from Table 3, addition of exogenous ThrABC activity aids in the production of threonine and glycine well above the level present in a wild-type *Methanococcus maripaludis.*

### EXAMPLE 6

### AMINO ACID EXPORT FROM HYDROGENOTROPHIC MICROORGANISMS

A candidate rhtA (threonine) or lysE (lysine) exporter gene from *M. maripaludis* is isolated and examined for functionality. Further mutations are optionally introduced to increase function, or the export gene is overexpressed by operably linking to a stronger promoter, or a functional exogenous rhtA or lysE gene is introduced into *M*. *maripaludis.* The overproduced aspartate pathway amino acids can be easily recovered and/or isolated from the culture medium.

### EXAMPLE 7

### ALTERING CARBON FLUX TO AMINO ACID PRODUCTION IN HYDROGENOTROPHIC MICROORGANISMS

The main carbon flow of a hydrogenotrophic microorganism (*e.g., M*. *maripaludis*) may be shifted in a variety of ways to provide more carbon to the aspartate amino acid biosynthesis pathways. For example, limiting the flow of carbon from pyruvate to phosphoenolpyruvate (PEP) is achieved by inactivating or down regulating the PEP synthase gene. In addition, the isoleucine pathway (if present) is optionally knocked out, which preserves pyruvate and acetyl CoA used by this pathway. Inactivation or reduction of particular enzymatic activities may be introduced by genetic engineering (*e.g*., gene or gene portion deletion) or by selecting for inactivation by mutation (*e.g*., spontaneous or induced). Alternatively, the pyruvate carboxylase gene is optionally overexpressed to funnel more carbon from pyruvate to oxaloacetate (OAA). In addition, the aspartate aminotransferase gene is optionally overexpressed to convert more OAA to aspartate. Overexpression may be accomplished, for example, by providing multiple copies of a gene or by altering the promoter region to provide stronger expression.

### EXAMPLE 8

### CULTURING NON-NATURAL AND RECOMBINANT HYDROGENOTROPHIC MICROORGANISMS

*M*. *maripaludis* is cultured in a bubble column bioreactor under anaerobic conditions for about 72 hours to 120 hours until the culture reaches a steady state condition, which be done in a series of consecutive vessels of increasing volume (e.g., starting at 50ml, using this culture to seed 10L, and then using this culture to seed 300L or more) so that a very large volume of dense culture is reached. During this time, the system will be running in a fed batch mode, wherein the syngas is continuously fed to the fermentation broth. The broth itself will not be exchanged. Once an appropriate OD₆₀₀ is reached (as measured by spectrophotometer), then a continuous culture process will be initiated, wherein an exchange of the media/broth is begun. The rate of exchange will be determined in view of the OD₆₀₀ of the culture within the fermenter. For example, from about1.5 to about 3.0 complete volumes of broth are exchanged per day.

The culture is maintained at a temperature of about 37°C, but might fluctuate in the range of about 35°C to about 40°C, maintained at a pH of about 7.0-7.2 (adjusting pH as needed with HCl and/or NaOH), and maintained at an OD₆₀₀ from about 1.5 to about 2.0. The syngas is comprised of H₂:CO₂ at a ratio ranging from about 4:1 to about 3:1, which may include carbon monoxide at a range of about 0% to about 5% (optimum is at most 1%), and may include other minor contaminants. The syngas flow rate is dictated by the specific design of the bubble or trickle column used in the process.

### EXAMPLE 9

### CULTURING NON-NATURAL AND RECOMBINANT HYDROGENOTROPHIC MICROORGANISMS WITH CO

Trel10-333UR was cultured in a 3.0L continuous stirred tank bioreactor under anaerobic conditions until the culture reached a steady state condition. The culture was started from a glycerol stock aliquot, which was used to inoculate 25mL of media contained in a 100ml sealed and anaerobic serum bottle, which are used as the seed culture for the reactor. The serum bottle was pressurized to 20 psig with a 4:1 ratio of H₂/CO₂ gas. Two such bottles are used to inoculate 1.5L of media that was being maintained at about 37°C and a pH of 7.0-7.2. The seed culture was allowed to grow for about 16 hours prior to being used to inoculate the main reactor. During this time, a 4:1 ratio of H₂/CO₂ was sparged into the main vessel. In addition, a constant agitation of 600 rpm was maintained using a combination of a Rushton and scoping blade. Approximately 8 hours prior to inoculation of the main vessel, a 200ppm bleed of H₂S gas was sparged into the main vessel. In addition, at the time of inoculation, a 1% (by volume) bleed of CO was also sparged into the main vessel. The culture reached a maximum OD within 48 to 56 hours, which is about the same amount of time needed for fermentation without CO to reach the same OD.

Fermentation titers of aspartate amino acids with Trel10-333UR at 56 hours without CO were 120mg/L glycine and 25.4 mg/L threonine, and with CO at 56 hours were 83mg/L glycine and 50mg/L threonine.

## Claims

1. A non-natural methanogenic archaea selected from,
(a) a non-natural *Methanococcus maripaludis* expressing a deregulated, aspartokinase mutant enzyme encoded by a recombinant *Methanococcus LysC* gene, wherein the aspartokinase mutant is resistant to feedback inhibition by lysine and/or threonine and has a mutation at the residue corresponding to residue G277, S302 or G359 in the aspartokinase encoded by *lysC* of *Corynebacterium glutamicum* ATCC 13032, or
(b) a non-natural *Methanococcus vannielii* expressing a deregulated, aspartokinase mutant enzyme encoded by a recombinant *Methanococcus LysC* gene that has a mutation at residue K380 or F339 and is resistant to feedback inhibition by lysine and/or threonine; and
wherein the non-natural methanogenic archaea (i) is not susceptible to growth inhibition by lysine and/or threonine, and (ii) metabolizes a H₂/COₓ substrate to produce one or more aspartate pathway amino acids at a higher level than a parent methanogenic archaea.

2. The non-natural methanogenic archaea of claim 1, wherein the non-natural methanogenic archaea is *Methanococcus maripaludis* and the deregulated aspartokinase mutant enzyme has a G to R mutation at the residue corresponding to G277, a S to P mutation at the residue corresponding to S302 or a G to E mutation at the residue corresponding to G359 in the aspartokinase encoded by *lysC* of *Corynebacterium glutamicum* ATCC 13032.

3. The non-natural methanogenic archaea of claim 2, wherein the deregulated aspartokinase mutant enzyme is a *Methanococcus maripaludis* aspartokinase with a mutation at residue G333.

4. The non-natural methanogenic archaea of claim 3, wherein the deregulated *Methanococcus maripaludis* aspartokinase has a G333R mutation.

5. The non-natural methanogenic archaea of claim 1, wherein the non-natural methanogenic archaea is *Methanococcus vannielii* and the deregulated aspartokinase mutant enzyme has a K380Q or a F339G mutation.

6. A method for producing an aspartate pathway amino acid, comprising culturing a non-natural methanogenic archaea according to any one of claims 1-5 for a time sufficient to produce one or more aspartate pathway amino acids.

7. The method of claim 6, wherein the aspartate pathway amino acid is lysine, threonine or methionine.

8. A system for producing an aspartate pathway amino acid, comprising:
(a) a source of gas comprising a H₂/COₓ substrate;
(b) a bioreactor comprising a non-natural methanogenic archaea according to any one of claims 1-5; and
(c) a connector disposed between the gas source and the bioreactor to allow flow of the gas into the bioreactor;
wherein the non-natural methanogenic archaea metabolizes the H₂/COₓ substrate to overproduce one or more aspartate pathway amino acids as compared to a parent methanogenic archaea;
wherein the bioreactor is optionally a liquid phase, bubble column, or trickle bed bioreactor and/or wherein the H₂/COₓ substrate is syngas or water-gas shifted syngas.

## Patentansprüche

1. Nicht-natürliches methanogenes Archaeon, ausgewählt aus
(a) einem nicht-natürlichen *Methanococcus maripaludis,* der ein durch ein rekombinantes *Methanococcus-LysC-*Gen codiertes dereguliertes Aspartatkinasemutanten-Enzym exprimiert, wobei die Aspartatkinasemutante resistent gegen Rückkopplungshemmung durch Lysin und/oder Threonin ist und an dem dem Rest G277, S302 oder G359 in der durch *LysC* von *Corynebacterium glutamicum* ATCC 13032 codierten Aspartatkinase entsprechenden Rest eine Mutation aufweist, oder
(b) einen nicht-natürlichen *Methanococcus vannielii,* der ein durch ein rekombinantes *Methanococcus-LysC-*Gen codiertes dereguliertes Aspartatkinasemutanten-Enzym exprimiert, das am Rest K380 oder F339 eine Mutation aufweist und resistent gegen Rückkopplungshemmung durch Lysin und/oder Threonin ist, und
wobei das nicht-natürliche methanogene Archaeon (i) nicht empfänglich für Wachstumshemmung durch Lysin und/oder Threonin ist und (ii) ein H₂/COₓ-Substrat metabolisiert, um eine oder mehrere Aminosäuren des Aspartat-Stoffwechselwegs auf einem höheren Niveau als ein methanogenes Stammarchaeon zu erzeugen.

2. Nicht-natürliches methanogenes Archaeon nach Anspruch 1, wobei es sich bei dem nicht-natürlichen methanogenen Archaeon um *Methanococcus maripaludis* handelt und das deregulierte Aspartatkinasemutanten-Enzym an dem G277 entsprechenden Rest eine G-zu-R-Mutation, an dem S302 entsprechenden Rest eine S-zu-P-Mutation oder an dem G359 entsprechenden Rest eine G-zu-E-Mutation in der durch LysC von *Corynebacterium glutamicum* ATCC 13032 codierten Aspartatkinase aufweist.

3. Nicht-natürliches methanogenes Archaeon nach Anspruch 2, wobei es sich bei dem deregulierten Aspartatkinasemutanten-Enzym um eine Aspartatkinase von *Methanococcus maripaludis* mit einer Mutation am Rest G333 handelt.

4. Nicht-natürliches methanogenes Archaeon nach Anspruch 3, wobei die deregulierte Aspartatkinase von *Methanococcus maripaludis* eine G333R-Mutation aufweist.

5. Nicht-natürliches methanogenes Archaeon nach Anspruch 1, wobei es sich bei dem nicht-natürlichen methanogenen Archaeon um *Methanococcus vannielii* handelt und das deregulierte Aspartatkinasemutanten-Enzym eine K380Q- oder eine F339G-Mutation aufweist.

6. Verfahren zum Erzeugen einer Aminosäure des Aspartat-Stoffwechselwegs, umfassend ein Kultivieren eines nicht-natürlichen methanogenen Archaeons nach einem der Ansprüche 1 bis 5 für eine ausreichende Zeit, um eine oder mehrere Aminosäuren des Aspartat-Stoffwechselwegs zu erzeugen.

7. Verfahren nach Anspruch 6, wobei es sich bei der Aminosäure des Aspartat-Stoffwechselwegs um Lysin, Threonin oder Methionin handelt.

8. System zum Erzeugen einer Aminosäure des Aspartat-Stoffwechselwegs, umfassend:
(a) eine ein H₂/COₓ-Substrat umfassende Gasquelle,
(b) einen ein nicht-natürliches methanogenes Archaeon nach einem der Ansprüche 1 bis 5 umfassenden Bioreaktor und
(c) einen zwischen der Gasquelle und dem Bioreaktor angeordneten Anschluss, um das Gas in den Bioreaktor strömen zu lassen,
wobei das nicht-natürliche methanogene Archaeon das H₂/COₓ-Substrat für eine Überproduktion einer oder mehrerer Aminosäuren des Aspartat-Stoffwechselwegs im Vergleich zu einem methanogenen Stammarchaeon metabolisiert,
wobei es sich bei dem Bioreaktor wahlweise um einen Flüssigphasen-, einen Blasensäulen- oder einen Rieselbett-Bioreaktor handelt und/oder wobei es sich bei dem H₂/COₓ-Substrat um Synthesegas oder durch Wassergas-Shift-Reaktion erhaltenes Synthesegas handelt.

## Revendications

1. Archée méthanogène non naturelle choisie parmi :
(a) un *Methanococcus maripaludis* non naturel exprimant une enzyme mutante d'aspartokinase dérégulée codée par un gène *Methanococcus LysC* recombinant, ladite mutante d'aspartokinase étant résistante à la rétroinhibition par la lysine et/ou la thréonine et comportant une mutation au niveau du résidu correspondant au résidu G277, S302 ou G359 dans l'aspartokinase codée par *lysC* de *Corynebacterium glutamicum* ATCC 13032, ou
(b) un *Methanococcus vannielii* non naturel exprimant une enzyme mutante d'aspartokinase dérégulée codée par un gène *Methanococcus LysC* recombinant qui comporte une mutation au niveau du résidu K380 ou F339 et est résistante à la rétroinhibition par la lysine et/ou la thréonine ; et
ladite archée méthanogène non naturelle (i) n'étant pas sensible à l'inhibition de croissance par la lysine et/ou la thréonine, et (ii) métabolisant un substrat H₂/COₓ pour produire un ou plusieurs acides aminés de la voie aspartate à un taux plus élevé qu'une archée méthanogène parente.

2. Archée méthanogène non naturelle selon la revendication 1, ladite archée méthanogène non naturelle étant *Methanococcus maripaludis* et ladite enzyme mutante d'aspartokinase dérégulée comportant une mutation G à R au niveau du résidu correspondant à G277, une mutation S à P au niveau du résidu correspondant à S302 ou une mutation G à E au niveau du résidu correspondant à G359 dans l'aspartokinase codée par *lysC* de *Corynebacterium glutamicum* ATCC 13032.

3. Archée méthanogène non naturelle selon la revendication 2, ladite enzyme mutante d'aspartokinase dérégulée étant une aspartokinase de *Methanococcus maripaludis* dotée d'une mutation au niveau du résidu G333.

4. Archée méthanogène non naturelle selon la revendication 3, ladite aspartokinase de *Methanococcus maripaludis* dérégulée comportant une mutation G333R.

5. Archée méthanogène non naturelle selon la revendication 1, ladite archée méthanogène non naturelle étant *Methanococcus vannielii* et ladite enzyme mutante d'aspartokinase dérégulée comportant une mutation K380Q ou une mutation F339G.

6. Procédé de production d'un acide aminé de la voie aspartate, comprenant la culture d'une archée méthanogène non naturelle selon l'une quelconque des revendications 1 à 5 pendant une durée suffisante pour produire un ou plusieurs acides aminés de la voie aspartate.

7. Procédé selon la revendication 6, ledit acide aminé de la voie aspartate étant la lysine, la thréonine ou la méthionine.

8. Système de production d'un acide aminé de la voie aspartate, comprenant :
(a) une source de gaz comprenant un substrat H₂/COₓ ;
(b) un bioréacteur comprenant une archée méthanogène non naturelle selon l'une quelconque des revendications 1 à 5 ; et
(c) un raccord disposé entre la source de gaz et le bioréacteur pour permettre l'écoulement du gaz dans le bioréacteur ;
ladite archée méthanogène non naturelle métabolisant le substrat H₂/COₓ pour surproduire un ou plusieurs acides aminés de la voie aspartate par rapport à une archée méthanogène parente ;
ledit bioréacteur étant éventuellement un bioréacteur en phase liquide, à colonne à bulle ou à lit ruisselant et/ou ledit substrat H₂/COₓ étant un gaz de synthèse ou un gaz de synthèse ayant subi une conversion eau-gaz.


### SEQUENCE LISTING

<110> Trelys, Inc.
   Bradshaw, Jill
   Hu, Zhihao
   Kouba, Jay
<120> COMPOSITIONS AND METHODS FOR BIOLOGICAL PRODUCTION OF AMINO ACIDS
<130> 910215.401WO
<140> US
   <141> 2015-01-02
<150> US 61/923,120
   <151> 2014-01-02
<160> 17
<170> FastSEQ for Windows Version 4.0
<210> 1
   <211> 19
   <212> DNA
   <213> Artificial Sequence
<220>
   <223> Forward primer
<400> 1
   gggacggcgc aacaaatgg 19
<210> 2
   <211> 23
   <212> DNA
   <213> Artificial Sequence
<220>
   <223> Reverse primer
<400> 2
   ggagatagtg agacccctgg agt 23
<210> 3
   <211> 36
   <212> DNA
   <213> Artificial Sequence
<220>
   <223> Primer sequence
<400> 3
   aaattatgag gcgcgcctcc ctgaagaaga agagag 36
<210> 4
   <211> 34
   <212> DNA
   <213> Artificial Sequence
<220>
   <223> Primer sequence
<400> 4
   tgcttattcg gcgcgccagt tccattttac cacc 34
<210> 5
   <211> 23
   <212> DNA
   <213> Artificial Sequence
<220>
   <223> Primer sequence
<400> 5
   caattactga acccaaagac cat 23
<210> 6
   <211> 23
   <212> DNA
   <213> Artificial Sequence
<220>
   <223> Primer sequence
<400> 6
   aatagttacc ggcgttacaa tca 23
<210> 7
   <211> 42
   <212> DNA
   <213> Artificial Sequence
<220>
   <223> Primer sequence
<400> 7
   aactaatagg tgaaacgcgt acaggaaaca cagaaaaaag cc 42
<210> 8
   <211> 46
   <212> DNA
   <213> Artificial Sequence
<220>
   <223> Primer sequence
<400> 8
   gatctcctag gcgcgcctca gactcctaac ttccatgaga gggtac 46
<210> 9
   <211> 51
   <212> DNA
   <213> Artificial Sequence
<220>
   <223> Primer sequence
<400> 9
   gatctcctag gcgcgcctta ctgatgattc atcatcaatt tacgcaacgc a 51
<210> 10
   <211> 47
   <212> DNA
   <213> Artificial Sequence
<220>
   <223> Primer sequence
<400> 10
   gatctcctag gcgcgcctta aaggtatttt tgaacgaata attcagc 47
<210> 11
   <211> 41
   <212> DNA
   <213> Artificial Sequence
<220>
   <223> Primer sequence
<400> 11
   catcagtaag gcgcgttttt atccaaaggt gaaagaatga a 41
<210> 12
   <211> 24
   <212> DNA
   <213> Artificial Sequence
<220>
   <223> Primer sequence
<400> 12
   tccctgatcg atagaaagtg tagt 24
<210> 13
   <211> 23
   <212> DNA
   <213> Artificial Sequence
<220>
   <223> Primer sequence
<400> 13
   ttgccgatga aattaaagtg aaa 23
<210> 14
   <211> 34
   <212> DNA
   <213> Artificial Sequence
<220>
   <223> Primer sequence
<400> 14
   gcgggcgcgc cgcataatta caccttatgc gttc 34
<210> 15
   <211> 33
   <212> DNA
   <213> Artificial Sequence
<220>
   <223> Primer sequence
<400> 15
   gcgggcgcgc ctaatcacgg ttcgtgatac tat 33
<210> 16
   <211> 40
   <212> DNA
   <213> Artificial Sequence
<220>
   <223> Primer sequence
<400> 16
   attacgccaa gcttggtacc actctcttct tcttcaggga 40
<210> 17
   <211> 40
   <212> DNA
   <213> Artificial Sequence
<220>
   <223> Primer sequence
<400> 17
   gtggatccga gctcggtacc tgagatcccc gcgctggagg 40